# EUROPEAN PATENT APPLICATION

(11) **EP 4 382 113 A1**
(43) Date of publication of application: **12.06.2024**
(21) Application number: 22838027.5
(22) Date of filing: 07.07.2022
(51) Int. Cl.: A61K 35/747, A61K 31/715, A61K 45/06, A61P 35/00

(54) **COMBINATION USE OF NOVEL LACTOBACILLUS PLANTARUM STRAIN AND STRAIN-DERIVED POLYSACCHARIDE FOR PREVENTING OR TREATING TUMORS**

(30) Priority: 09.07.2021 KR 20210090328
(71) Applicant: Immunobiome Inc., Nam-gu Pohang-si, Gyeongsangbuk-do 37666 (KR); POSTECH Research and Business Development Foundation, Pohang-si, Gyeongsangbuk-do 37673 (KR)
(72) Inventor: IM, Sin-Hyeog, Pohang-si Gyeongsangbuk-do 37660 (KR); SHARMA, Garima, Pohang-si Gyeongsangbuk-do 37666 (KR); SHARMA, Amit Kumar, Pohang-si Gyeongsangbuk-do 37666 (KR); PARK, Sunhee, Pohang-si Gyeongsangbuk-do 37662 (KR)
(74) Representative: Mewburn Ellis LLP
(86) International application number: PCT/KR2022/009884
(87) International publication number: WO 2023/282675

(57) **Abstract**

The present invention relates to a combination use of a novel *Lactobacillus plantarum* IMB19 strain and/or polysaccharides derived from the strain with an anticancer drug for preventing or treating tumors. The novel Lactobacillus plantarum IMB19 strain and polysaccharides derived from the strain of the present invention exhibit an excellent ability to stimulate CD8+T cell activity and exhibit excellent Treg cell inhibitory activity, and stimulate and improve antitumor immune response through various mechanisms such as increased macrophage infiltration in CPS tumors and differentiation and reprogramming of macrophages into an inflammatory phenotype (M1). Furthermore, the strain and the polysaccharides may significantly increase antitumor immune response when administered in combination with an anticancer drug, particularly an immuno-anticancer drug such as a PD-1 or PD-L1 inhibitor (e.g., a PD-1 antibody, a PD-L1 antibody, or the like), and exhibit a remarkable tumor growth suppression effect compared to the case of using the anticancer drug alone. Therefore, the present invention can be effectively used to prevent, relieve, or treat tumors through administration in combination with various anticancer drugs.

## Description

### Technical Field

The present invention relates to the use of co-administration of novel *Lactobacillus plantarum* IMB19 strain KCTC 14337BP and/or a polysaccharide derived from the strain with an anticancer agent for the prevention or treatment of tumors, and more specifically, to the use of *Lactobacillus plantarum* IMB19 strain KCTC 14337BP having immune-stimulation and anti-tumor activity or the polysaccharide of Formula I derived from the strain in combination with an anticancer agent for the prevention or treatment of tumors.

### Background Art

Mammals possess an array of microbes that constantly interact with the immune system. Commensal microorganisms form a symbiotic relationship with hosts and interact with the hosts in various processes such as digestion, behaviors, and maturation of the immune system (Cerf-Bensussan N, Gaboriau-Routhiau V. The immune system and the gut microbiota: friends or foes? Nat Rev Immunol 2010;10(10):735-44.). Likewise, fungi are present in the human body and affect the immune system of hosts (Wheeler ML, Limon JJ, Underhill DM. Immunity to Commensal Fungi: Detente and Disease. Annu Rev Pathol 2017;12:359-85.). Innate immune cells detect various pathogen-associated molecular patterns (PAMPs) on the surface of fungal cells, including polysaccharides, through pattern recognition receptors (PRRs), such as Toll-like receptors (TLRs). Upon detection of the signal, innate immune cells alter the gene expression profiles and produce immune signaling molecules such as cytokines to modulate adaptive immunity (Iliev ID, Leonardi I. Nat Rev Immunol 2017;17(10) :635-46., Underhill DM, Iliev ID. Nat Rev Immunol 2014; 14(6) :405-16. and Brubaker SW, Bonham KS, Zanoni I, et al. Annu Rev Immunol 2015;33:257-90.).

The WHO defines probiotics as live microorganisms that are beneficial to the health of the host when administered in appropriate amounts (Nat Rev Gastroenterol Hepatol 11, 506-514 (2014)). Probiotics has been reported to act as a supplement to the gut microflora of the host and to be effective in improving intestinal barrier function and modulating the immune system of the host (Microb Ecol Health Dis 26, 25877 (2015)). Most probiotics belong to the phylum *Firmicutes*, which is the largest single bacterial phylum, and most are Gram-positive bacteria with low "G+C" content, and are mainly classified into the Bacilli and Clostridia groups. *Lactobacillus* species are lactic acid bacteria (LAB) belonging to the family *Lactobacillaceae. Lactobacillus* is a well-known microbial family with a distinct ecological status. Several lactic acid bacteria are known to be traditionally associated with foods such as milk, dairy products, fermented foods, and sausages. Lactobacilli are a group of microorganisms generally regarded as safe (GRAS) by the FDA and are widely used in food and other industries. Lactobacilli are classified as facultatively anaerobic, non-spore-forming, non-motile, rod-shaped, and gram-positive bacteria, and are generally catalase-negative. Lactobacillus may be homofermentative or heterofermentative, and produce lactic acid as a final product of primary fermentation (Front Cell Infect Microbiol 2, 86 (2012)). Lactobacillus exhibits smooth and convex colonies.

Molecular identification methods are needed to identify individual strains due to the similar biochemical and morphological characteristics between LABs. Several lactobacilli have been isolated and characterized from foods, particularly fermented foods. Fermentation generally refers to biochemical changes attributable to microorganisms. Kimchi, a traditional Korean food, is mainly fermented cabbage and shows beneficial effects in terms of nutritional health (Crit Rev Food Sci Nutr 34, 175-203 (1994)). Kimchi is known to have a unique microbial community. Various reports show that lactic acid bacteria are mainly present in kimchi, and *Weisellia*, *Lactobacillus*, and *Leuconostoc* are dominant species, and in particular, *Lactobacillus plantarum* strains are known to be the dominant species (Food Sci Biotechnol 19, 641-646 (2010)). *Lactobacillus plantarum* is one of the most studied strains due to the strain-specific probiotic profile and technological applications in the food industry. Most of the kimchi microbiome is culturable (Int J Food Microbiol 102, 143-150 (2005)) and isolation of individual microbes is essential to the study of the health benefits thereof.

Meanwhile, the growth and proliferation of cancer is strictly regulated by the host immune response. In order to grow and proliferate tumor cells, the tumor cells must be able to evade the surveillance of the immune system, which induces early death of tumor cells. Tumor cells grow and proliferate by evading the immune system by formation of an immunosuppressive tumor microenvironment through various pathways such as secretion of cytokines and expression of cell surface molecules. As a cancer treatment strategy targeting these immune evasion mechanisms, various efforts to induce or strengthen the immune system have been attempted. Tumor immunotherapy may refer to a treatment method for restoring or enhancing the ability of the immune system to recognize or destroy tumors in order to overcome immunosuppression or immune evasion mechanisms acquired by tumors. In 2011, ipilimumab was an immunotherapeutic agent that successfully treated patients with malignant melanoma. Since then, various immunotherapies such as nivolumab and pembrolizumab have been continuously developed.

Reports and cases showing the importance of gut microbiota as a tool for such tumor immunotherapy and applications thereof are increasing. The gut microbiome plays an essential role in forming the local and systemic immune responses of the host (Science 330, 1768-1773 (2010), Cell 148, 1258-1270 (2012)). The diversity and configuration of the gut microbiome has also been shown to affect responsiveness to chemotherapy (Cancer Immunol Immunother 55, 1470-1479 (2006); Science 342, 971-976 (2013)). In particular, certain commensal microorganisms have been found to be associated with activation of spontaneous anti-tumor immunity, and experiments (Science 350, 1084-1089 (2015), Science 350, 1079-1084 (2015)) and human cancer (Science 359, 91-97 (2018), Nature 453, 620-625 (2008)) are known to exhibit a synergistic effect on the therapeutic efficacy of immunotherapeutic agents. Thus, it has clearly found that certain strains, such as gut microbiome, can also affect extramucosal and distant tumor progression. Based on these various reports, alteration of the gut microbiome is considered as an option for effective and feasible clinical treatment. Currently, most of the studies have demonstrated the comprehensive effects of a specific strain or population of strains on the immune system of the host or antitumor immunity in terms of probiotics, but information about active ingredients derived from specific strains that exhibit these effects and mechanisms such as signaling pathways thereof has not been revealed.

Combination therapy is a therapeutic strategy for several infectious, autoimmune, neurological, and cardiovascular diseases, in addition to cancer, and is a classical approach that combines at least one therapeutic agent or therapy to provide better efficacy than single therapeutic agent or therapy. In general, drugs having orthogonal activity have been combined for the treatment of cancer, but currently, a combination of drugs or therapies having additional or synergistic effects is required to increase efficacy and reduce side effects.

Targeted therapies including chemotherapy and small molecule inhibitors have been the main clinical methods for treating tumors in the clinic for the past half century, but immune checkpoint inhibitors have recently emerged as more effective treatment options. Immune checkpoint inhibitors (ICIs) are applicable to various tumors and exhibit sustained reactivity, minimal toxic effects, and excellent clinical results and tolerability.

However, one of the biggest drawbacks of immune checkpoint inhibitors is the limited response of ICIs in the parent population. Although various mechanisms may act due to factors related to non-responsive populations and patient genomics or acquired resistance, it has recently been reported that the gut commensal microbial community influences the responsiveness of patients to immunotherapy. FMTs in donors who respond to ICIs have been shown to improve objective responses of non-responsive patients. The role of individual gut microbes in enhancing anticancer effects was first recognized when the enhancement of the effects of *Bacteroides fragilis* and *Bifidobacterium* on anti-CTLA-4 and anti-PD-L1 was reported. Since then, various bacterial strains show similar effects, but different efficiencies, so a combination of immune checkpoint inhibitors and strains with better efficiency is needed.

Under this technical background, as a result of extensive efforts to clearly determine the correlation and mechanism between the components, structure, molecular weight, and the like of polysaccharides derived from microorganisms and immune modulatory functions, the present inventors identified a novel strain belonging to *Lactobacillus plantarum* having a remarkably high immune enhancing activity from kimchi, a traditional Korean food, and found that the capsular polysaccharide of the novel strain, and a fraction having a specific structure thereof (CPS-100) were effective molecules exhibiting immune stimulating and enhancing effects. Specifically, the novel strain and novel polysaccharides with specific structures can greatly inhibit tumor growth by stimulating the immune response based on various mechanisms, such as activation of CD8+ T cell function, increased macrophage infiltration in CPS tumors, and differentiation/reprogramming of macrophages into inflammatory phenotypes, and filed a patent application based thereon (Korean Patent Application Nos. 2021-0003432 and 2021-0003433).

Furthermore, as a result of intensive efforts to develop a cancer treatment strategy through combination of various conventional therapeutic uses for cancer and the strain or polysaccharide derived from the strain, the novel strain greatly improved the anti-tumor immune effects by stimulating the cytotoxic T cell activity through antigen-presenting cells, when *Lactobacillus plantarum* IMB19 strain and various immuno-cancer agents, such as anti-PD-L1 antibodies, are co-administered and completed the present invention based thereon.

The information disclosed in this Background section is provided only for enhancement of understanding of the background of the present invention, and therefore it may not include information that forms the prior art that is already obvious to those skilled in the art.

### Summary of the Invention

Therefore, it is one object of the present invention to provide the use of co-administration of a novel strain having immune-stimulating activity and an anticancer drug.

It is another object of the present invention to provide the use of co-administration of polysaccharide derived from the strain having immune-stimulating activity and an anticancer drug.

It is another object of the present invention to provide an immune regulation method through co-administration of the strain and/or polysaccharide derived from the strain and an anticancer agent, and/or a method of preventing, ameliorating or treating tumor or an infectious disease.

In accordance with one aspect of the present invention, the above and other objects can be accomplished by the provision of a composition for preventing or treating tumors containing a *Lactobacillus plantarum* IMB19 strain KCTC14337BP, the composition being administrated in combination with an anticancer agent.

In accordance with another aspect of the present invention, provided is a composition for preventing or treating tumors containing a *Lactobacillus plantarum* IMB19 strain KCTC 14337, and an anticancer agent.

In accordance with another aspect of the present invention, provided is a composition for preventing or treating tumors containing a polysaccharide represented by the following Formula I as an active ingredient, the composition being administered in combination with an anticancer agent:

[Formula I] -[-D-B-I-F-G-C-E-H-A-]ₙ-

wherein
A and D are galactose;
B, C, E, G and H are rhamnose;
F is N-acetylglucosamine;
I is glucose; and
n is an integer from 1 to 10.

In accordance with another aspect of the present invention, provided is a composition for preventing or treating tumors containing a polysaccharide represented by the following formula I and an anticancer agent:

[Formula I] -[-D-B-I-F-G-C-E-H-A-]ₙ-

wherein
A and D are galactose;
B, C, E, G and H are rhamnose;
F is N-acetylglucosamine;
I is glucose; and
n is an integer from 1 to 10.

In accordance with another aspect of the present invention, provided is the use of co-administration of the *Lactobacillus plantarum* IMB19 strain with an anticancer agent for the prevention, amelioration or treatment of tumors.

In accordance with another aspect of the present invention, provided is the use of co-administration of the polysaccharide of Formula 1 with an anticancer agent for the prevention, amelioration or treatment of tumors.

In accordance with another aspect of the present invention, provided is a method for preventing, ameliorating or treating tumors including co-administering the *Lactobacillus plantarum* IMB19 strain and an anticancer agent to a subject.

In accordance with another aspect of the present invention, provided is a method for preventing, ameliorating or treating tumors including co-administering the polysaccharide of Formula I and an anticancer agent to a subject.

In accordance with another aspect of the present invention, provided is the use of the strain and/or polysaccharide for the preparation of a composition for co-administration for preventing or treating tumors or infectious diseases.

### Brief Description of Drawings

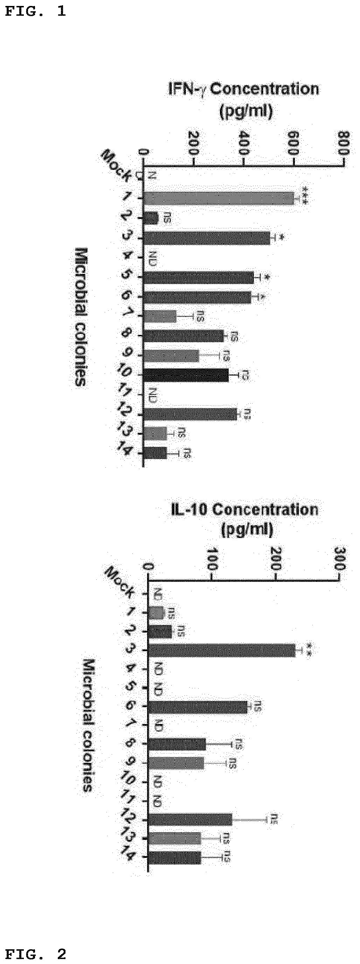

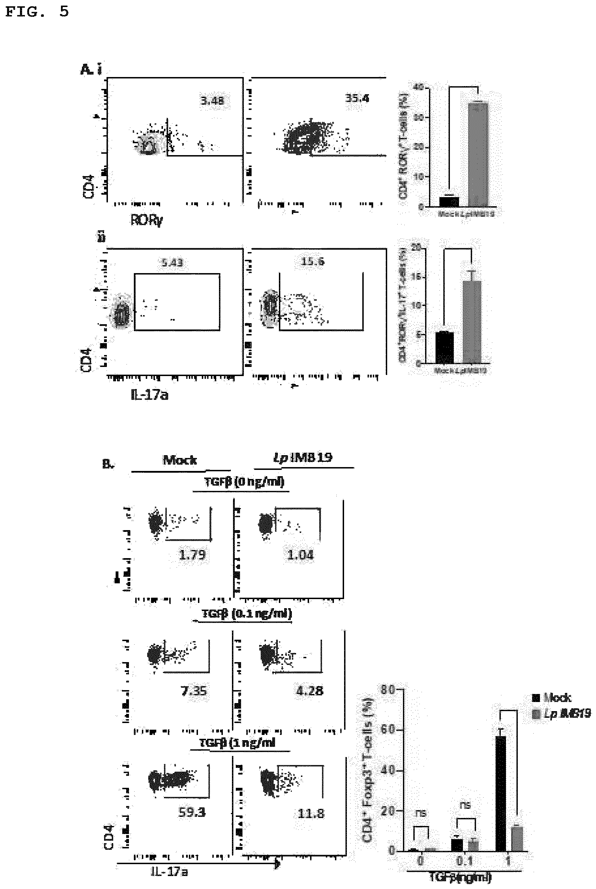

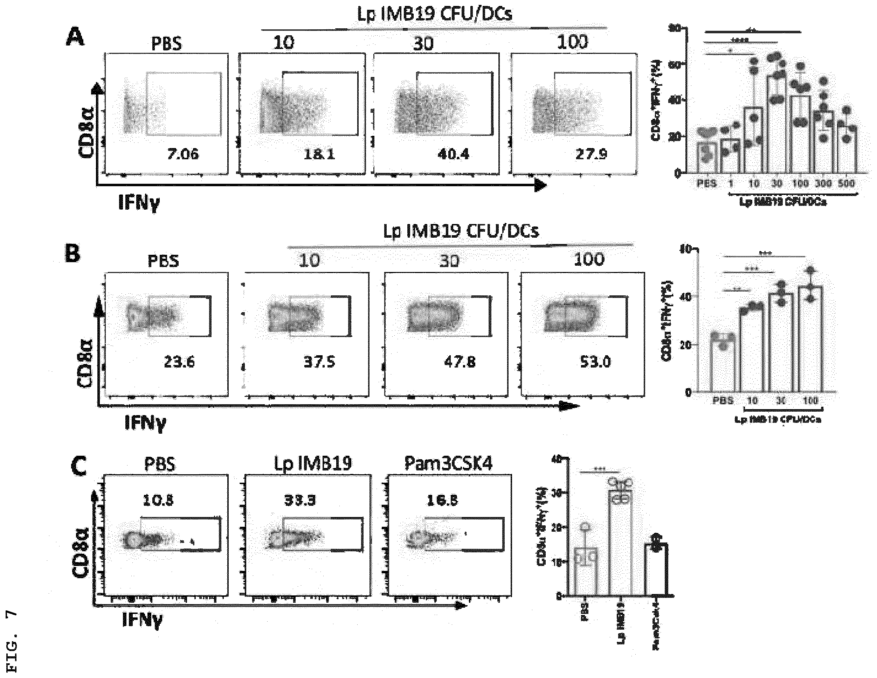

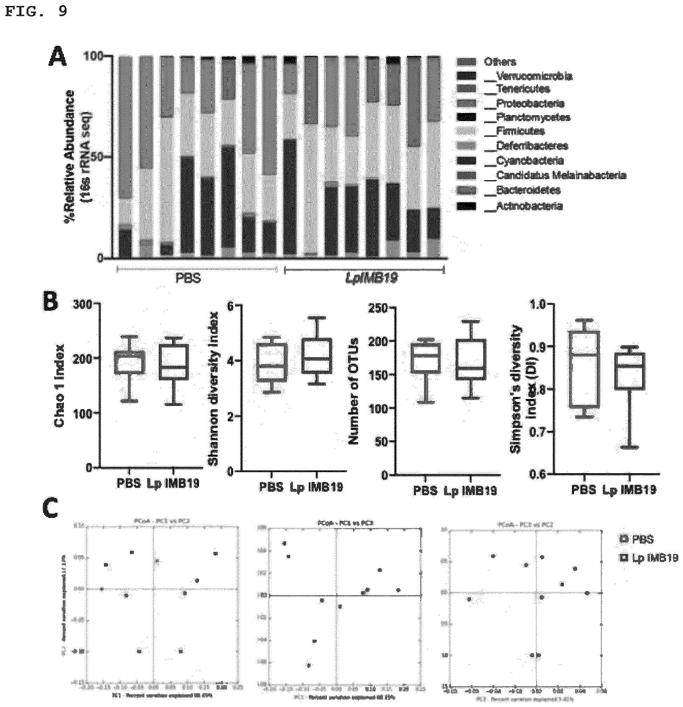

FIG. 1 shows the levels of inflammatory or anti-inflammatory cytokines in spleen cells when treated with kimchi-derived strains.
   Dilutions of kimchi suspension were prepared, streaked onto MRS-agar plates and cultured at 37°C for 48 hours, and then strains were obtained from 14 colonies and further cultured in MRS broth for 24 hours. Splenocytes and each strain were mixed at a ratio of 1:10 and cultured for 48 hours at 37°C and 5% CO₂ conditions. Cytokine levels in the culture supernatant were measured by ELISA to evaluate the activation of splenocytes. The data are expressed as mean ± SEM (n = 2). Statistical significance was calculated for Mock by one-way ANOVA. *p<0.5, **p<0.01, ***p<0.001
FIG. 2 illustrates the microbiological and biological properties of *L. plantarum* IMB19 strain.
FIG. 2A shows a TEM image of the *L. plantarum* IMB19 strain. Arrows indicate the capsular layer around the cell wall.
FIG. 2B shows hemolytic activity of the *L. plantarum* IMB19 strain after culturing for 48 hours in 5% sheep blood agar, positive control: *Bacillus cereus* ATCC 27348
FIG. 2C shows gelatin hydrolysis test for *L. plantarum* IMB19 strain. Positive control: *B. cereus* ATCC11778
FIG. 3 shows the cluster dendrogram of *L. plantarum* IMB19.
   A phylogenetic tree was constructed based on the differences between *L. plantarum* strains. The average nucleotide index (ANI) was calculated using the OrthANI algorithm. The distance is directly proportional to the difference in genome.
FIG. 4 shows the Th 17 cells production of various *Lactobacillus plantarum* strains.

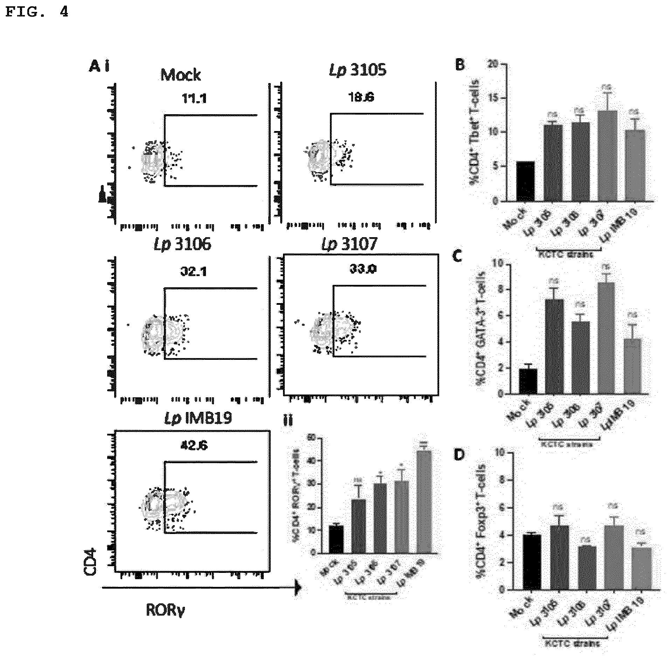

FIG. 4 shows flow cytometry data showing the effects of *L*. *plantarum* IMB19, *L. plantarum* 3105, *L. plantarum* 3106, and *L. plantarum* 3107 on naive CD4+ T cells. Dendritic cells primed with *L. plantarum* IMB19 were cultured along with naive CD4 + T cells, anti-CD3 (10 ng/ml) and IL-2 (100 U/ml).
FIG. 4A i is a FACS plot illustrating the production of Th17 cells. The cells were gated as live CD4+RORγT cells.
FIG. 4A ii is a bar graph showing the average cell number for Th17 cells gated as live CD4+ RORγT cells.
FIG. 4B shows Th1 cells gated as live CD4+ T-bet+ T cells.
FIG. 4C shows Th2 cells gated as live CD4+ GATA3+ T cells.
FIG. 4D is Treg gated as live CD4+ Foxp3+ regulatory T cells.
   Data are expressed as mean ± SD and statistical significance was calculated for Mock by one-way ANOVA. **p<0.01
FIG. 5 shows the ability of *L. plantarum* IMB19 to suppress Treg and induce Th17 cells.
FIG. 5A i is a representative FACS plot and bar graph showing the effects of *L. plantarum* IMB19 on the production of Th17 cells. The dendritic cells primed with *L. plantarum* IMB19 were co-cultured with naive CD4+ T cells, anti-CD3 (0.1 ug/ml), TGF-β (0.5 ng/ml), IL-6 (2 ng/ml), IL1-βIL-2 (100 U/ml), anti-IL4 (10 µg/ml) and anti-IFNγ (10 ug/ml). The cells were gated as live CD4 + RORγT cells.
FIG. 5A ii is a representative FACS plot and bar graph showing the level of interleukin-17 in Th17 cells produced with *L*. *plantarum* IMB19. The cells were gated as live CD4+ RORγT cells.
FIG. 5B is a representative FACS plot and bar graph showing the inhibitory effect of *L. plantarum* IMB19 on Treg cell production *in vitro.* The dendritic cells primed with *L. plantarum* IMB19 were co-cultured along with naive CD4+ T cells, anti-CD3 (0.1 ug/ml), IL-2 (100 U/ml) and various concentrations of TGF-β. Data are expressed as mean ± SEM. Statistical significance was analyzed by ordinary one-way ANOVA. **p < 0.01, ***p < 0.001

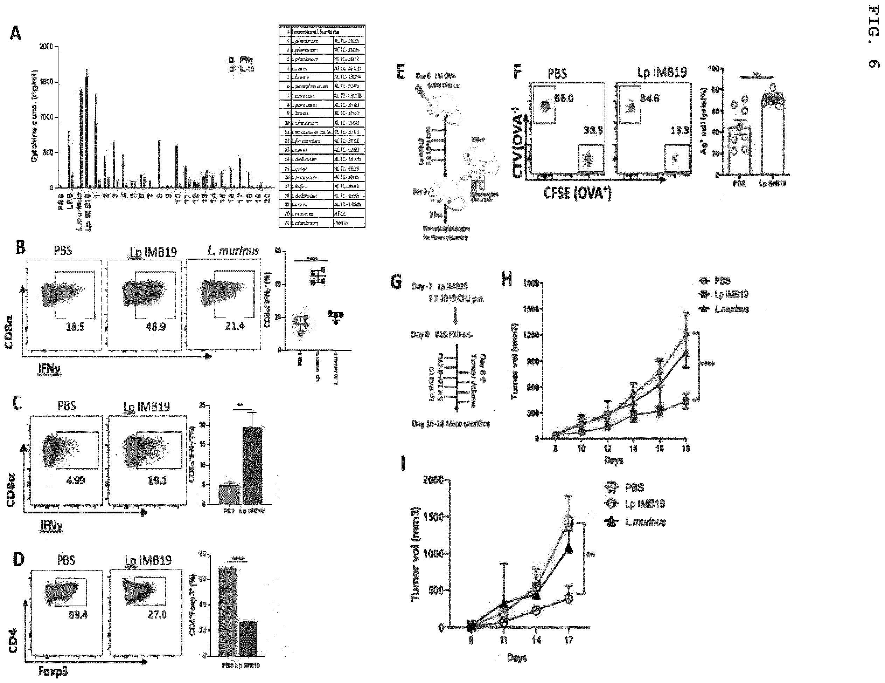

FIG. 6 shows the anti-tumor immune response through CD8+ T cell activation enhanced by *L. plantarum* IMB19.
FIG. 6A shows the result of cytokine analysis through ELISA of the splenocyte-bacteria co-culture supernatant.
FIG. 6B shows the result of quantification of IFNγ cells from the co-culture of splenic CD11c+ dendritic cells and naive CD8+ T cells primed with *L. plantarum* IMB19 or *L. murinus.*
FIG. 6C shows the result of quantification of IFNγ cells from the co-culture of naive CD8+ T cells and CD11b+F4/80+ peritoneal macrophages primed with *L. plantarum* IMB19.
FIG. 6D shows the result of quantification of Foxp3+CD4+ T cells from the co-culture of splenic CD11c+ DCs primed with *L*. *plantarum* IMB19, naive CD4+ T cells, and 2 ng/ml of TGFβ.
   The data are mean ± SEM and B was analyzed by ordinary one-way ANOVA with Tukey's multiple comparisons. **p < 0.01, ****p < 0.0001
FIG. 6E is a schematic diagram illustrating the *in vivo* OVA-expressing Listeria monocytogenes (LM-OVA) cytotoxicity assay.
FIG. 6F shows *L. plantarum* IMB19-mediated CD8+ T cell-specific cytotoxicity against OVA+ splenocytes in mice infected with LM-OVA.
FIG. 6G is a schematic diagram illustrating the *in vivo* B16.F10 melanoma mouse model.
FIG. 6H shows B116.F10 melanoma growth kinetics in C57/BI6 germ-free mice treated or not treated with *L. plantarum* IMB19 or *L. murinus.*
FIG. 6I shows B116.F10 melanoma growth kinetics in C57/BI6 SPF mice treated or not treated with *L. plantarum* IMB19 or *L. murinus.*
   The data are expressed as mean ± SEM, FIG. 6F shows the result of analysis by non-parametric, two tailed t-test and FIGS. 6I show two-way ANOVA with Dunnet's multiple comparisons. **P<0.01, ***P<0.001, ****P<0.0001
FIG. 7 shows the dose-dependent effect of *L. plantarum* IMB19 on CD8 + T cells.
FIGS. 7A and 7B show the result of quantification of IFNγ cells in the co-culture of naive CD8+ T cells with spleen (7A) or mLN (7B) CD11c+ dendritic cells primed with different ratios of *L*. *plantarum* IMB19.
FIG. 7C shows the result of quantification of IFNγ cells in the co-culture of naive pmel TCR-transplanted CD8+ T cells with splenic CD11c+ dendritic cells primed with *L. plantarum* IMB19 at different ratios in the presence of 100 ng/ml of gp-100. Data are expressed as means ± SEM and were analyzed by ordinary one-way ANOVA with Tukey's multiple comparisons. **p <0.01, ***p <0.001, ****p <0.0001

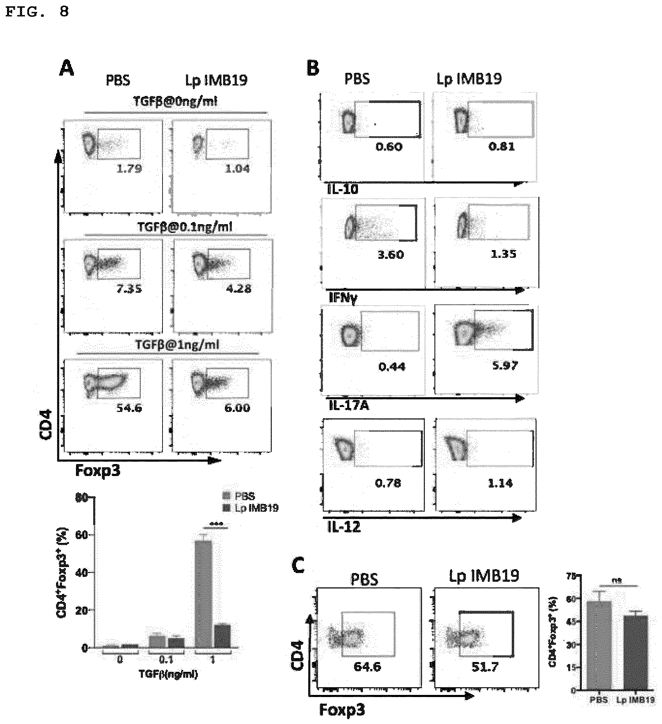

FIG. 8 shows the growth inhibition of regulatory T-cells through the production of interleukin-6 by *L. plantarum* IMB19.
FIG. 8A shows the result of quantification of Foxp3 + CD4+ T cells in the co-culture of splenic CD11c+ dendric cells primed with *L. plantarum* IMB19 and naive CD4+ T cells at different TGFβ concentrations.
FIG. 8B shows the result of quantification of Foxp3 + CD4+ T cells in the co-culture of splenic CD11c+ dendric cells primed with *L. plantarum* IMB19 and naive CD4+ T cells at a TGFβ concentration of 2 ng/ml.
FIG. 8C shows the result of quantification of Foxp3+ CD4+ T cells in the co-culture of splenic CD11c+ dendric cells primed with *L. plantarum* IMB19 and naive CD4+ T cells at 0.01 ng/ml of TGFβ and IL-6 -/- splenic DC.
Data are expressed as means ± SEM and were analyzed by ordinary one-way ANOVA with Tukey's multiple comparisons. ***p <0.001, ns: not significant
FIG. 9 shows the effect of *L. plantarum* IMB19 on tumor immunity without changing microbial diversity.
FIG. 9A shows the result of phylogenetic analysis of phyla (%) in fecal samples of tumor-bearing mice fed *L. plantarum* IMB19 in FIG. 6H.
FIGS. 9B and 9C show alpha diversity analysis (B) and principal coordinates analysis of bacterial β diversity in fecal samples of tumor-bearing mice fed *L. plantarum* IMB19 in FIG. 6H. Data represent values obtained from duplicate independent experiments.

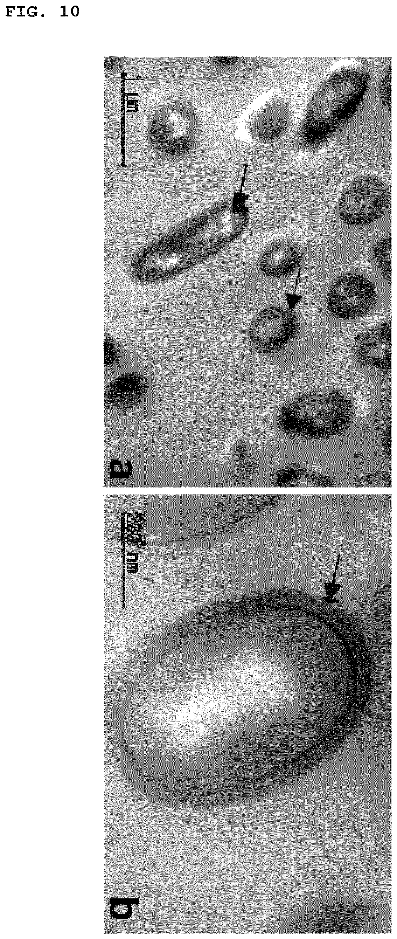

FIG. 10 is a transmission electron microscope (TEM) image illustrating *L. plantarum* IMB19.
FIG. 10A shows the *L. plantarum* IMB19 bacterial community, and arrows represent individual bacteria.
FIG. 10B shows *L. plantarum IMB19* and the arrow represents a thick capsular layer around the cell wall.

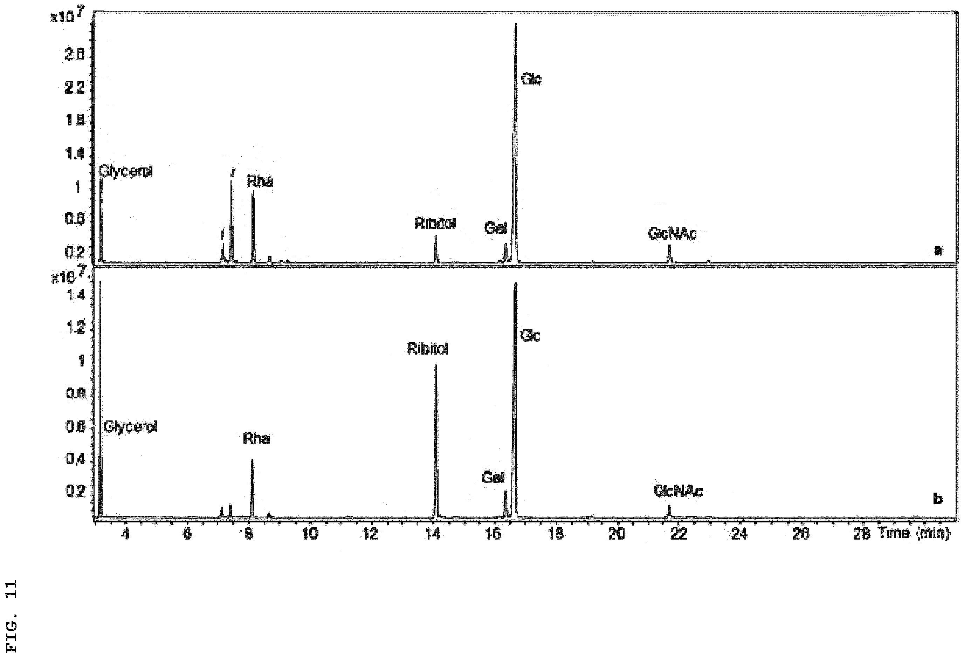

FIG. 11 shows GC-MS profiles of acetylated methylglycosides derived from CPS extracted from *L. plantarum* IMB19 dephosphorylated samples without (a) or with (b) HF treatment. "i" represents an impurity.

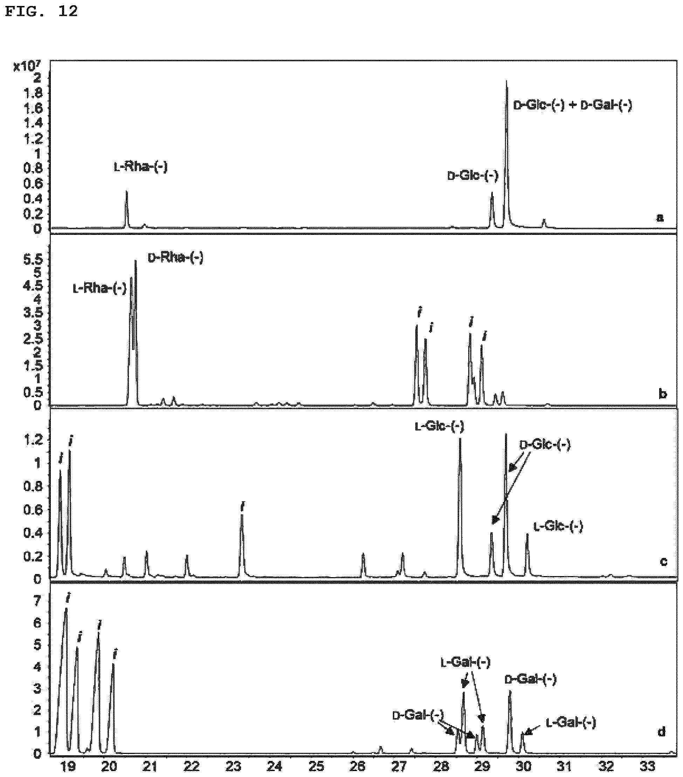

FIG. 12 shows acetylated 2-(-)-octyl derivatives (a), rhamnose (b), glucose (c), and galactose acetylated 2-(-)-octyl glycoside standard (d) of CPS extracted from *L. plantarum* IMB19 GC-MS profile. *"*i*"* represents an impurity.

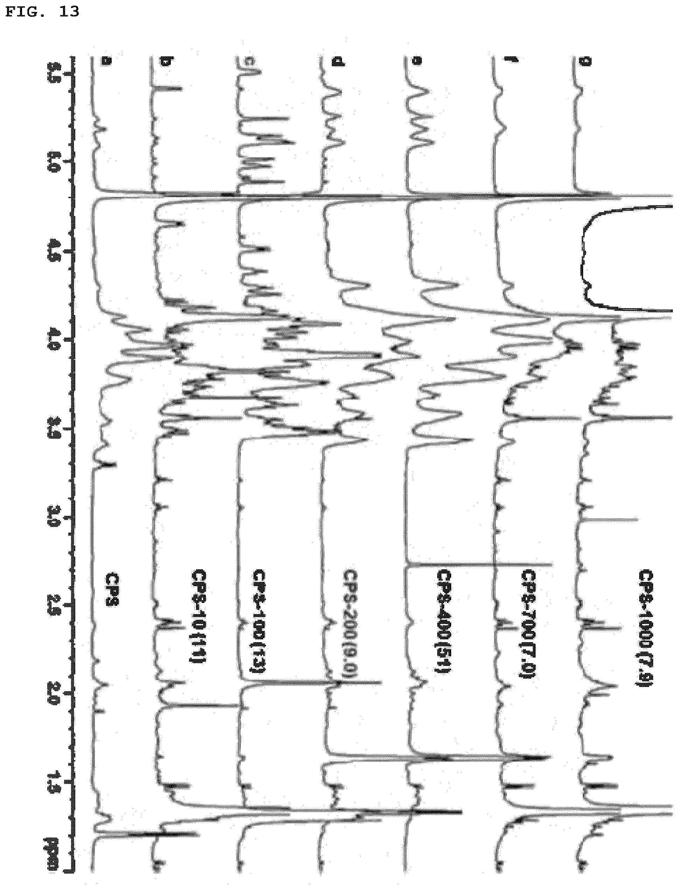

FIG. 13 shows proton spectra of fractions obtained by purifying crude CPS through ion exchange chromatography (600 MHz, 298 K): CPS (a), CPS-10 (b), CPS-100 (c), CPS- 200 (d), CPS-400 (e), CPS-700 (f) and CPS7-1000 (g). The numbers in parentheses indicate the yield (mg/mg) of each fraction based on 28 mg of crude CPS.

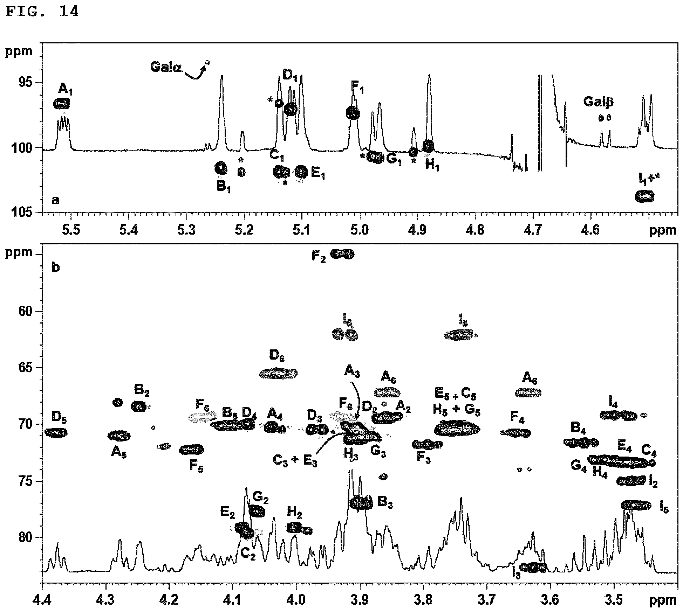

FIG. 14 shows the expansion of the HSQC spectrum recorded for the capsular polysaccharide of *L. plantarum* IMB19 (600 MHz, 310 K): (a) expansion of the anomeric region; and (b) expansion of the carbinolic area. The gray densities correspond to *"*CH₂*"* carbons and *"***"* indicates reduced forms (the minor anomeric signals of the sugars of repeating units attached to galactose with Galα and Galβ).

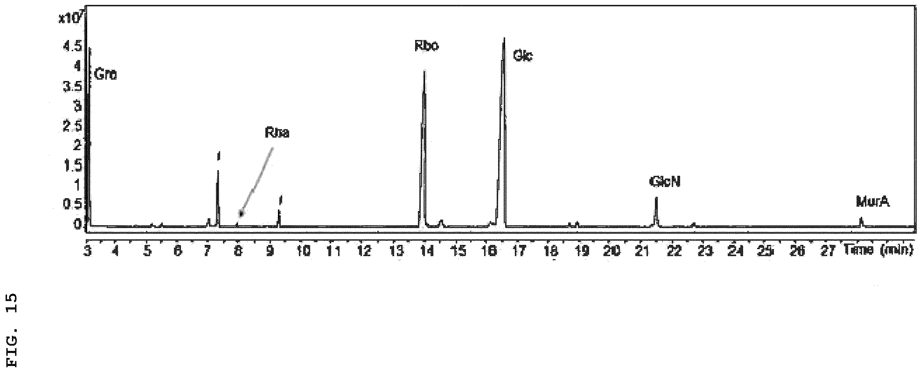

FIG. 15 shows a GC-MS profile of acetylated methylglycoside of CPS-400 extracted from *L. plantarum* IMB19 after the sample was dephosphorylated by treatment with HF. *"*i*"* represents an impurity and MurA represents muramic acid, a component of bacterial peptidoglycan.

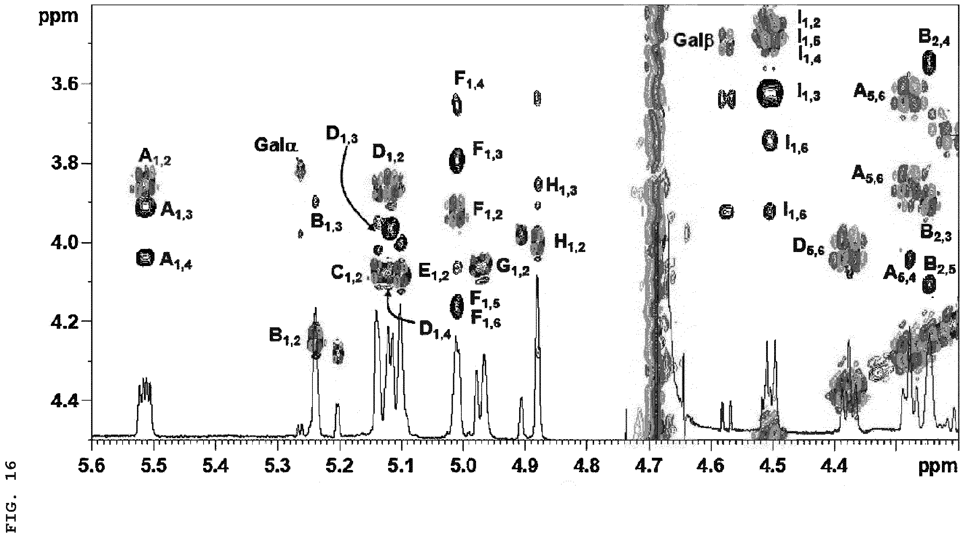

FIG. 16 shows the expansion of the TOCSU (black) and COZY (cyan/red) spectra recorded for the capsular polysaccharide of *L*. *plantarum* IMB19, CPS-100 (600 MHz, 310 K).

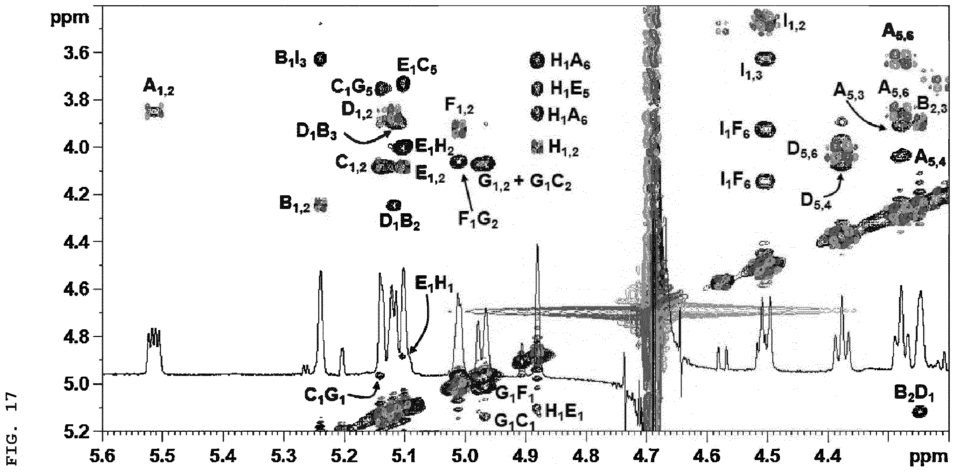

FIG. 17 shows the expansion of the NOESY (black) and COZY (cyan/red) spectra recorded for the capsular polysaccharide of *L*. *plantarum* IMB19, CPS-100 (600 MHz, 310K).

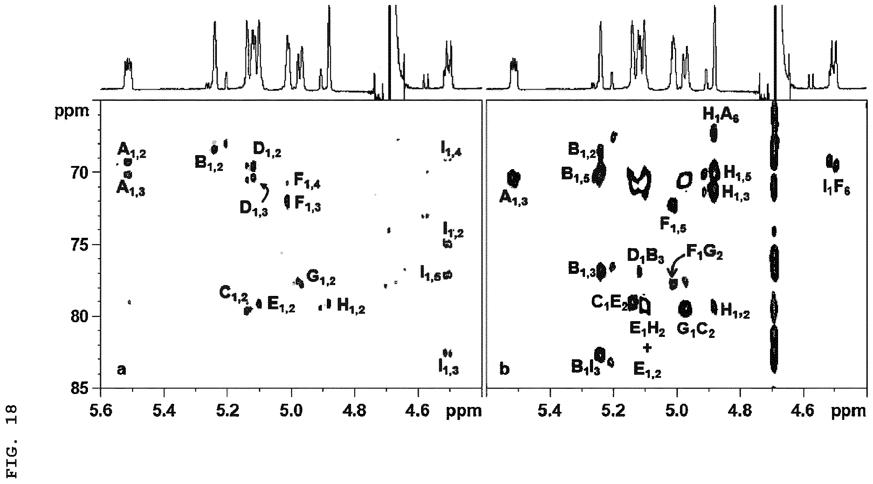

FIG. 18 shows the expansion of the HSQC-TOCSY (a) and HMBC (b) NMR spectra recorded for the capsular polysaccharide of *L*. *plantarum* IMB19 (600 MHz, 310 K).

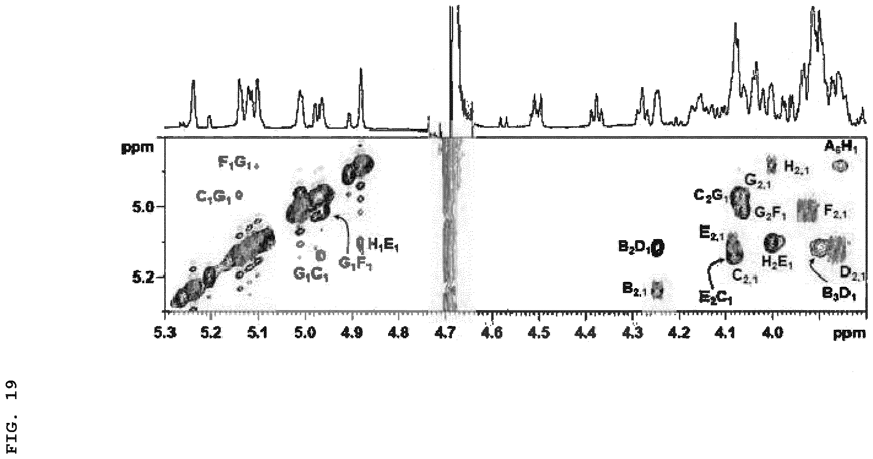

FIG. 19 shows the expansion of NESY (black) and COZY (cyan/red) spectra specifically illustrating the H-2 proton region of Zangin B-G, which is the part of the capsular polysaccharide of *L. plantarum* IMB19, CPS-100 (600 MHz, 310K).

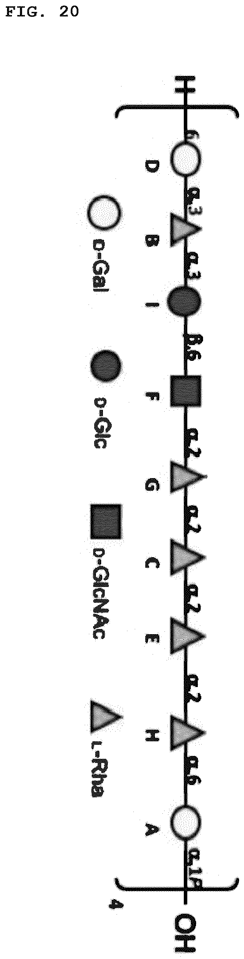

FIG. 20 shows the structure of the repeating unit of CPS-100, a capsular polysaccharide of *L. plantarum* IMB19. 4 is the calculated average degree of polymerization. The phosphoric acid group (P) that substituted the oxygen of the 6' carbon residue of D represents a phosphodiester linkage between A and D of the repeating units of the polymerized polysaccharide by bonding to the 1' carbon of A.

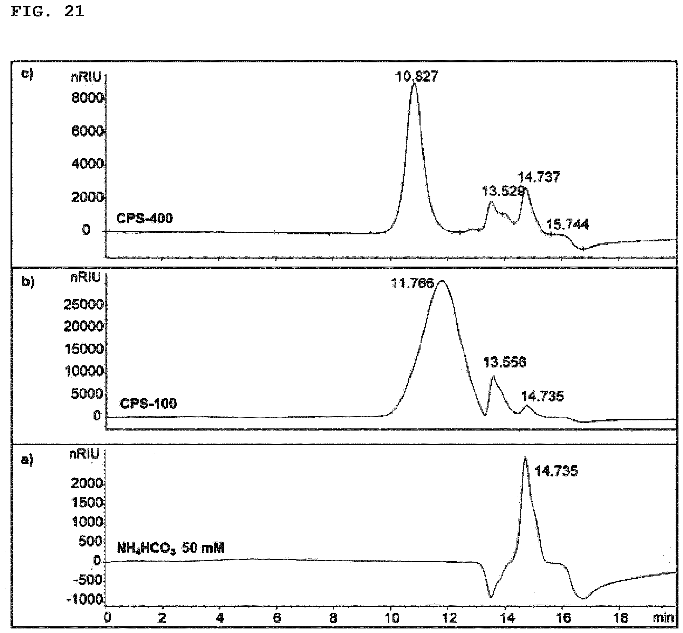

FIG. 21 shows the HPSEC profile of single solvent injection (a); CPS-100 (b); and CPS-400(c). The peaks at 13.6 and 14.74 minutes are solvent-induced artifacts as can be seen from the profile (a) of the single solvent injection.

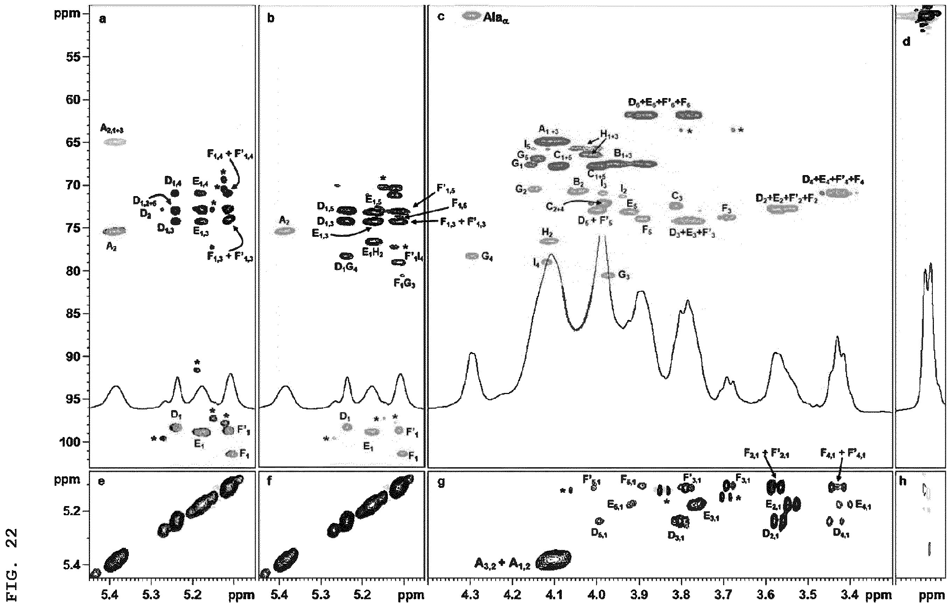

FIG. 22 shows NMR spectra recorded for CPS-400 (600 MHz, 310K): (a) overlay of HSQC-TOCSY (black) and HSQC (cyan); (b) overlay of HMBC (black) and HSQC (cyan); TOCSY spectra of (c) HSQC; (d) HSQC-TOCSY; and (e) to (h) different regions. "*" indicates density belonging to an unidentified minor motif. Labeling and depiction of the structural units are shown in Table 4.

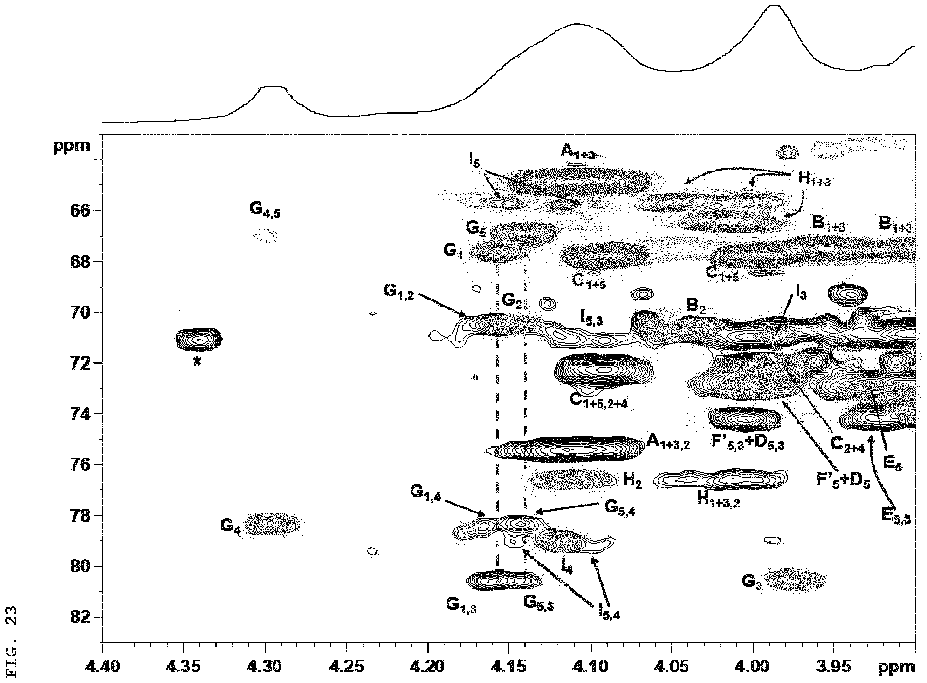

FIG. 23 shows the expansion of the HSQC-TOCSY (black/gray) and HSQC (cyan/red) showing the density of Ribitol G. HSQC-TOCSY correlations from G1 and G5 are demonstrated by gray dotted lines. Labeling and description of the structural units are shown in Table 4 (600 MHz, 310K).

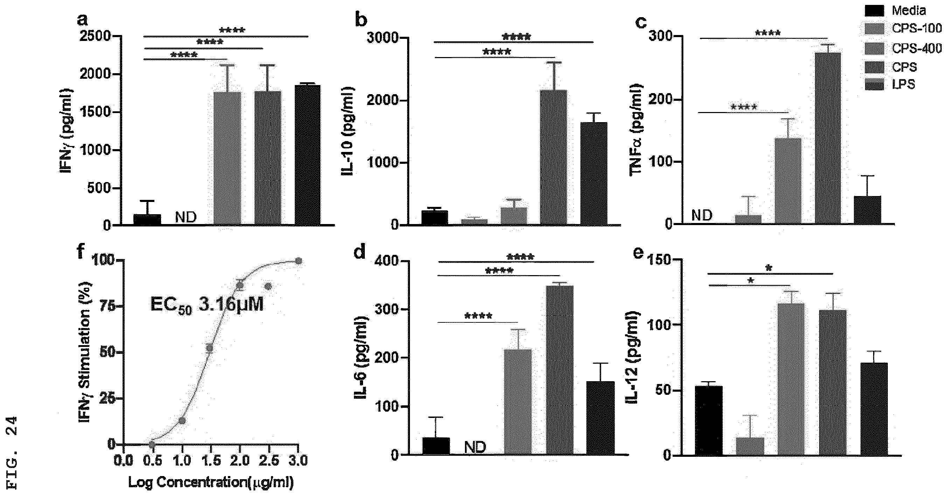

FIG. 24 shows the levels of cytokines to determine the immune-stimulating activity of the capsular polysaccharide of the purified fraction. Splenocytes are cultured in the presence of medium (control), CPS fraction (CPS-400 and CPS-100, 50 pg/mL), total CPS (50 pg/mL), and lipopolysaccharide (LPS from *E. Coli* 0111:B4, 0.1 pg/mL), as shown in FIG. 24. Cell culture supernatants were analyzed by ELISA to evaluate cytokine production. Dose response curve of CPS-100 for production of (a) IFNγIL-10; (c) TNF-α; (d) IL-6; (e) IL-12; and (f) IFNγ. In (f), the half maximal effective concentration (EC₅₀) was calculated to be 3.16 µM. Data are values obtained from 2 to 3 independent experiments with similar results, and all bar graphs represent mean ± SD. * p<0.05, **** p<0.0001 (one-way ANOVA using post hoc Dunnet's test for multiple comparisons); ND, not detected.

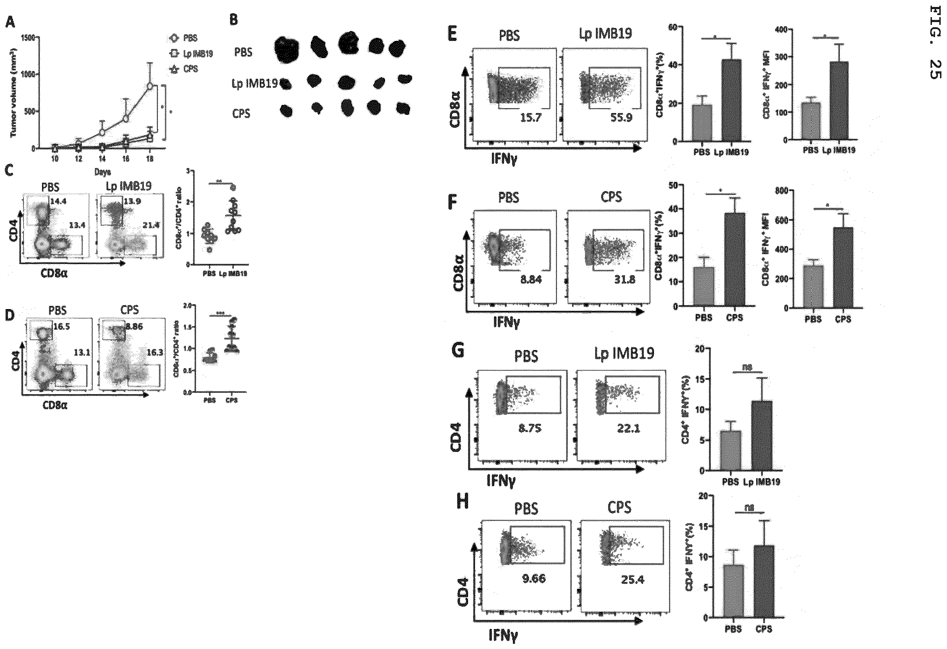

FIG. 25 shows the tumor growth inhibitory effects of *L*. *plantarum* IMB 19 and CPS through activation of CD8+ T cells and enhancement of tumor invasion.
FIG. 25A shows B16.F10 melanoma growth kinetics in C57/Bi6 SPF mice treated or not treated with *L. plantarum* IMB 19 or CPS.
FIG. 25B is an image showing tumors isolated from mice.
FIGS. 25C and 25D show the percentage of tumor-infiltrating CD8+ and CD4+ T cells determined by flow cytometry 16 to 18 days after the start of *L. plantarum* IMB 19 (C) or CPS (D) treatment.
   Data are expressed as mean ± SEM. Data were analyzed by two-way ANOVA (A) or non-parametric, two tailed t-test (C-D) with Dunnet's multiple comparisons. *p < 0.05, **p < 0.01, ***p < 0.001.
FIGS. 25E and 25F show the percentage of tumor-infiltrating IFNγ cells and the mean fluorescence intensity (MFI) of tumor-infiltrating CD8+ T cells when treated or not treated with *L*. *plantarum* IMB 19 (E) or CPS (F). FIGS. 25G to 25H show the frequency of IFNγ cells when treated or not treated with *L. plantarum* IMB 19 (G) or CPS (H). Data are expressed as mean ± SEM. Data were analyzed with a non-parametric, two tailed t-test. *P<0.05, ns: not significant.

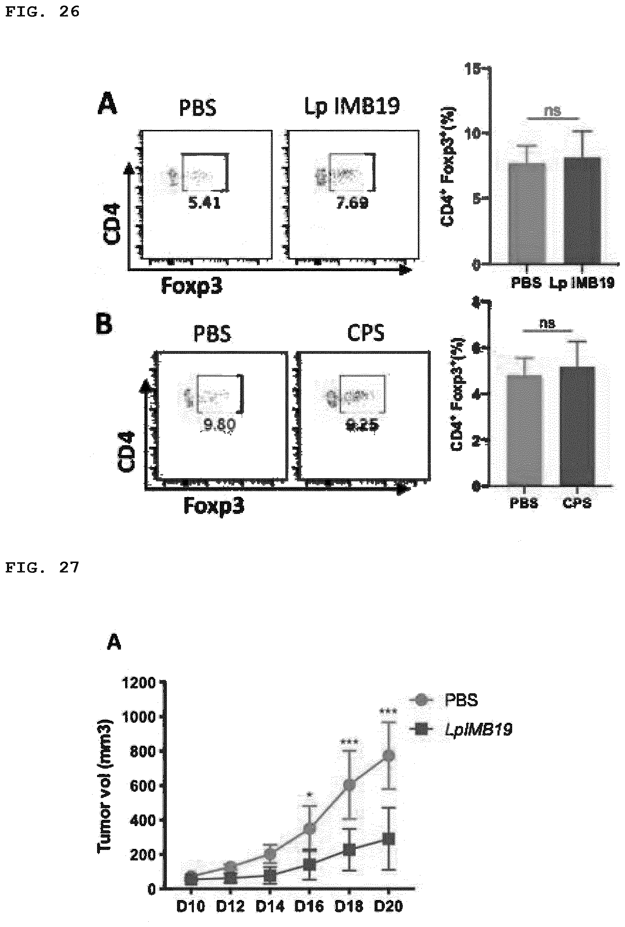

FIG. 26 shows that *L. plantarum* IMB19 and CPS do not alter intratumoral regulatory T cell populations.
FIGS. 26A and 26B show the percentage of tumor-infiltrating CD4+Foxp3+ regulatory T cells in tumor-infiltrating lymphocytes when treated with *L. plantarum* IMB 19 (A) or CPS (B). Data were analyzed with a non-parametric, two tailed t-test. *P<0.05, ns: not significant.
FIG. 27 shows the tumor growth inhibitory activity of *L*. *plantarum* IMB19 in EMT breast cancer.
FIG. 27A shows EMT-6 breast cancer growth kinetics in Balb/c SPF mice treated with or not treated with *L. plantarum* IMB19. Data are expressed as means ± SEM and were analyzed by two-way ANOVA with Dunnet's multiple comparisons. *P<0.05, ***P<0.001.

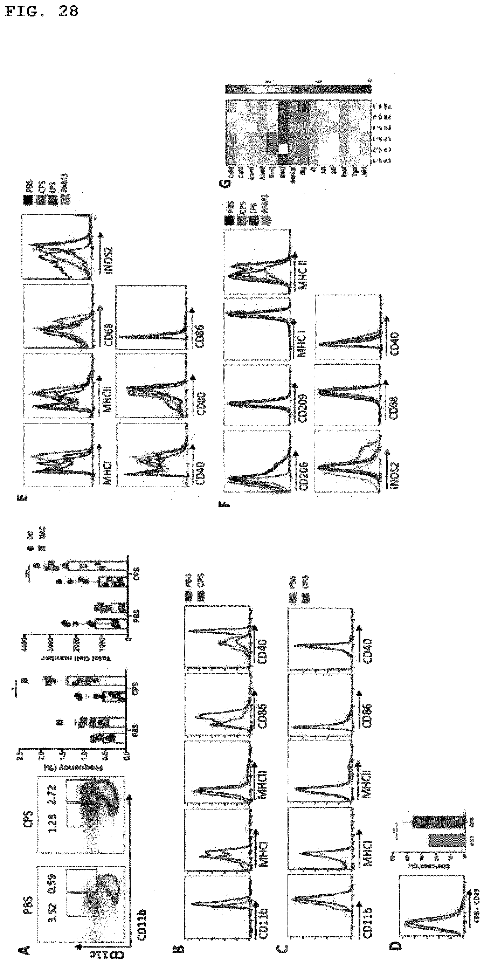

FIG. 28 shows the intratumoral infiltration-enhancing activity of CPS in inflammatory macrophages in B16.F10 melanoma of CPS.
FIG. 28A shows the number and percentage of CD45+CD11c+CD11b+ tumor-infiltrating macrophages upon CPS treatment 40 hours after tumor inoculation.
FIGS. 28B and 28C show active markers, CD11b, MHC I, MHC II, CD86 and CD40, on tumor-infiltrating macrophages (B) and dendritic cells (C) upon CPS treatment.
FIG. 28D shows the percentage of CD8+CD69+ T cells in tumor draining lymph nodes upon CPS treatment. Data are expressed as means ± SEM and were analyzed by two-way ANOVA (A) or non-parametric, two tailed t-test (B) with Dunnet's multiple comparisons *P<0.05, **P <0.01, ***P<0.001.
FIGS. 28E and 28F show active markers, MHC I, MHC II, iNOS2, and CD68 of mouse peritoneal macrophages ex *vivo* treated with CPS (10 µg/ml) immediately after isolation (E) or after IL-4 treatment (F) for 24 hours.
FIG. 28G shows the result of David pathway analysis of the enriched M1 macrophage gene signature in CPS- or PBS-treated tumor-derived macrophages isolated 40 hours after tumor inoculation.

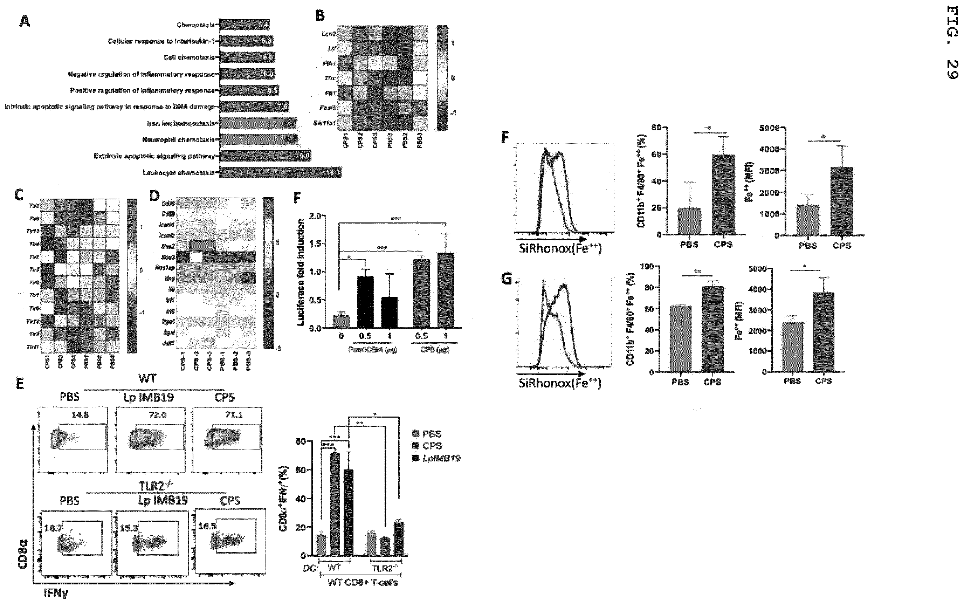

FIG. 29 shows that tumor growth can be inhibited through the action of CPS as a ligand of TLR2 and the sequestration of iron in macrophages.
FIG. 29A shows biological pathways and functions within a subset of genes elevated levels in each macrophage evaluated by David pathway analysis after isolating the initially infiltrated macrophages from the tumors of CPS- and PBS-administered mice and performing mRNA sequencing for gene expression profiling (fold change ≥1.5).
FIGS. 29B to 29D are heat maps illustrating the relative abundance of genes showing significant changes among genes related to iron sequestration (B), TLR (C), and inflammatory macrophage markers (D).
FIG. 29E shows the results of co-culture of CPS (10 ug/ml)- and LpIMB19-primed wild-type or TLR2 -/- splenic CD11c + dendritic cells and wild-type mouse-derived naive CD8+ T cells, followed by quantification of IFN-γT cells.
FIG. 29F shows the activity evaluated by SEAP secretion in the culture supernatant by QUANTI-BLUE SEAP reporter assay after culturing HEK-TLR2 cells along with the TLR2 ligand Pam3Csk4 or CPS for 24 days. In FIGS. 29E to 29F, data are expressed as mean ± SD analyzed by two-way ANOVA with Dunnett's multiple comparison. *<0.05, **P<0.01, ***P<0.001.
FIG. 29F shows fluorescence intensity and an average thereof of iron (Fe⁺⁺) in CD45+CD11c+CD11b+F4/80+ tumor-infiltrating macrophages 18-20 days after transplantation of B16.F10 melanoma.
FIG. 29G shows the result of quantification of the iron intake 30 minutes after exposure to iron to the culture medium depending on CPS treatment. Data are mean ± SD and are analyzed by non-parametric, two tailed t-test, and ns indicates not significant.

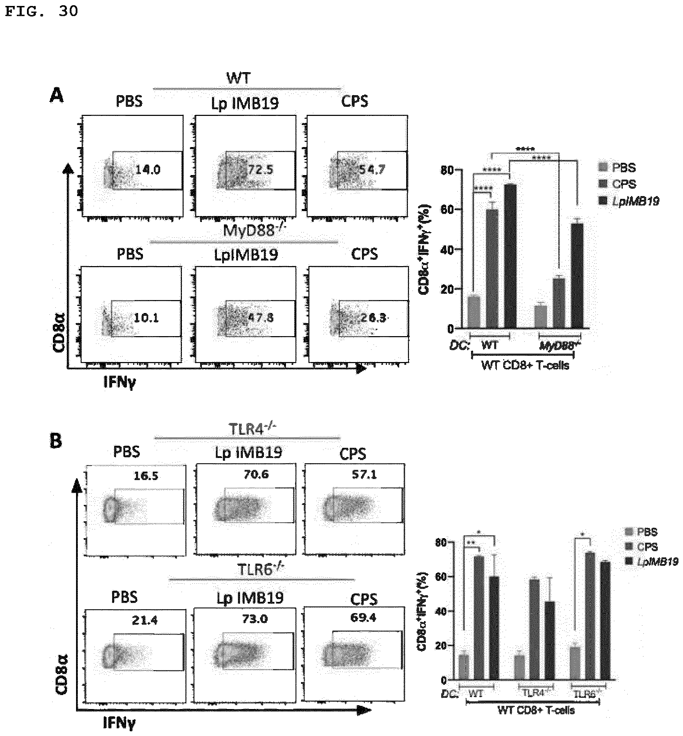

FIG. 30 shows CPS activity mediated by TLRs on APC and shows the result of quantification of IFN-γT cells when CPS (10 µg/ml)- and LpIMB19-primed wild-type, MyD88-/-, TLR4-/-, or TLR6-/- splenic CD11c+ dendritic cells and naive CD8+ T cells derived from wild-type mice were co-cultured. Data are mean ± SD and were analyzed by two-way ANOVA with Dunnett's multiple comparison. *<0.05, **P<0.01, ****P<0.0001.

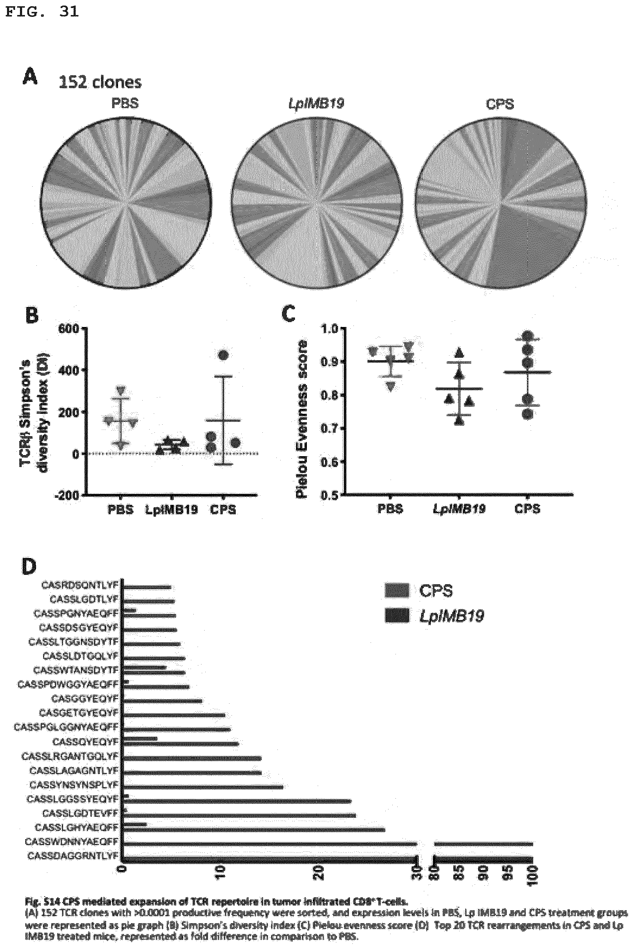

FIG. 31 shows CPS-mediated expansion of the TCR repertoire in tumor-infiltrating CD8 + T cells. FIG. 31A is a pie graph showing 152 TCR clones with a production frequency of >0.0001 and expression levels in PBS, CPS (10 µg/ml) and LpIMB19-treated groups. FIG. 31B shows Simpson's Index, FIG. 31C shows the Pielou uniformity score, and FIG. 31D shows the top 20 TCR rearrangements in mice treated with PBS, CPS (10 µg/ml) and LpIMB19 as fold difference in comparison to PBS.

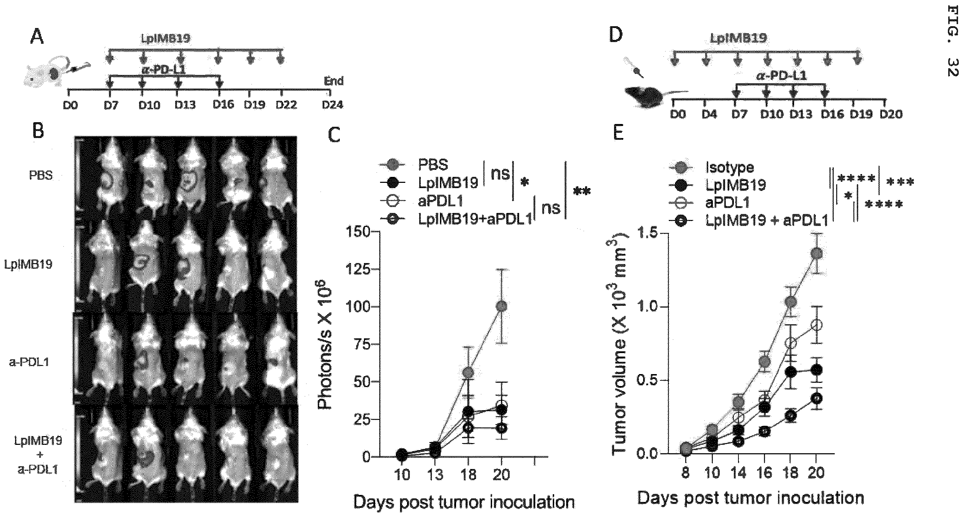

FIG. 32 shows the significant tumor growth inhibitory effect of LpIMB19 in combination with an immune checkpoint inhibitor, anti-PD-L1. Mice were treated with i) PBS (isotype), ii) LpIMB19 (1×10⁹ CFU/mouse p.o), iii) anti-PD-L1 (100 ug/mouse i.p.) and a combination of ii) and iii). FIGS. 32A to 31C show Renca-Luc orthotopic mouse renal cancer models. Tumor cells (0.04 mill/mouse) were surgically implanted under the mouse kidney capsule, and mice were randomized on day 7 based on bioluminescence imaging.
FIG. 32A is a schematic diagram illustrating the therapy.
FIG. 32B shows representative *in vivo* bioluminescence images (BLI) at day 20, wherein BLI was performed on days 10, 13, 18, 20 and 24 to follow the growth of renal cancer.
FIG. 32C shows longitudinal BLI showing tumor progression.
FIG. 32D is a schematic diagram illustrating co-administration to a B16.F10 mouse melanoma model.
FIG. 32E shows tumor progression in melanoma. B16.F10 cells (0.2 milli/mouse) were subcutaneously injected and subjected to various therapies, and tumor progression was tracked over 20 days. Mean ± SEM is expressed and analyzed by two-way analysis of variance (ANOVA). *P < 0.05, **P < 0.001, ***P < 0.0001, ****P < 0.00001.

### Detailed Description and Preferred Embodiments of the Invention

Unless defined otherwise, all technical and scientific terms used herein have the same meanings as appreciated by those skilled in the field to which the present invention pertains. In general, the nomenclature used herein is well-known in the art and is ordinarily used.

In one embodiment of the present invention, the present inventors identified a novel *Lactobacillus plantarum* IMB19 strain having high immune-stimulating activity from kimchi, a traditional fermented food in Korea, and deposited the strain in the Korea Center for Biological Resources (Accession number: KCTC 14337BP) .

In another embodiment of the present invention, in a co-culture system designed to include both the innate and adaptive immune systems, the strain is capable of increasing immune stimulatory cytokines (e.g., IFN-γ), inhibiting anti-inflammatory cytokines (e.g., IL-10), remarkably increasing effector T cells, and inhibiting Treg production, thereby stimulating the immune system in the host and inhibiting tumor growth and proliferation.

In another embodiment of the present invention, the present inventors found that co-administration of the strain and the immune checkpoint inhibitor greatly improved antitumor effects and greatly inhibited the tumor growth compared to single administration of the strain or the immune checkpoint inhibitor.

In one aspect, the present invention is directed to a composition for preventing or treating tumors containing a *Lactobacillus plantarum* IMB19 strain KCTC14337BP, the composition being administered in combination with an anticancer agent.

In the present invention, the *Lactobacillus plantarum* IMB19 strain KCTC14337BP may exhibit excellent immune-enhancing activity and antitumor activity.

More specifically, the strains of the present invention exhibit various immune-enhancing activities and/or anti-tumor activities of i) inducing T cell differentiation in an inflammatory direction, such as induction of helper T cells and inhibition of Treg cell differentiation, ii) stimulating CD8+ T cells, enhancing the activity thereof and improving intratumoral infiltration, iii) inhibiting Treg cell activity, iv) increasing intratumoral macrophage infiltration, and v) activating macrophages into inflammatory cells and reprogramming M2 to M1 macrophages.

In the present invention, the *Lactobacillus plantarum* IMB19 strain (hereinafter abbreviated as L. plantarum IMB19) was isolated from kimchi, and was identified as a novel strain by 16S rRNA analysis, recA amplification/band comparison, whole gene sequencing, and phylogenetic, morphological and physiological analysis, and then deposited.

In the present invention, the *L. plantarum* IMB19 strain may be derived from fermented foods, for example, kimchi.

In the present invention, the *L. plantarum* IMB19 strain may have small, smooth, and circular translucent colonies.

In the present invention, the *L. plantarum* IMB19 strain may be non-flagellated.

In the present invention, the *L. plantarum* IMB19 strain may have rod-shaped colonies.

In the present invention, the *L. plantarum* IMB19 strain may have a capsular layer.

In the present invention, the *L. plantarum* IMB19 strain may be non-hemolytic (non-) or γ hemolytic (γ).

In the present invention, the *L. plantarum* IMB19 strain may be negative for gelatinase activity.

In the present invention, the *L. plantarum* IMB19 strain does not produce biogenic amines such as histamine, cadaverine, tyramine and/or putrescine.

In the present invention, the *L. plantarum* IMB19 strain may have resistance to kanamycin.

In the present invention, the *L. plantarum* IMB19 strain may have immunity-enhancing and/or antitumor activities. As a more specific example, the *L. plantarum* IMB19 strain exhibits various immune-enhancing activities and/or anti-tumor activities of i) inducing T cell differentiation in an inflammatory direction, such as induction of helper T cells and inhibition of Treg cell differentiation, ii) stimulating CD8+ T cells, enhancing the activity thereof and improving intratumoral infiltration, iii) inhibiting Treg cell activity, iv) increasing intratumoral macrophage infiltration, and v) activating macrophages into inflammatory cells and reprogramming M2 to M1 macrophages, but is not limited thereto.

In the present invention, the *L. plantarum* IMB19 strain may inhibit tumor growth.

In the present invention, the anti-cancer agent may be, for example, a chemo-anticancer agent (cytotoxic anti-cancer agent), a targeted anti-cancer agent, an immuno-anticancer agent, a hormonal anti-cancer agent, a cell therapy agent, or the like, but is not limited thereto.

In the present invention, the chemical anticancer agent is also called a *"*cytotoxic anticancer agent*"*, and is an anticancer agent that directly attacks cancer cells and exhibits an anticancer effect, and examples thereof include, but are not limited to, alkylating agents such as cyclophosphamide, ifosfamide, bendamustin, melphalan, and cisplatin; metabolic antagonists such as capecitabine, cytarabine, doxifluridine, fluorouracil, clofarabine, fludarabine and decitabine; DNA topoisomerase inhibitors such as doxorubicin, daunorubicin, idarubicin, etoposide and topotecan; microtubule inhibitors such as cabazitaxel, docetaxel, and vincristine; and other chemotherapeutic agents such as mitomycin C, bleomycin, and hydroxyurea.

In the present invention, the target anticancer agent is an anticancer agent containing, as a main component, a substance that binds to or interacts with a specific antigen expressed by cancer or a cancer-related antigen as a target, and examples thereof include antibody therapeutics such as cetuximab, trastuzumab, bevacizumab, rituximab, ibritumomab, alemtuzumab, brentuximab, and elotuzumab; and signaling inhibitors such as erlotinib, gefitinib, vandetanib, afatinib, lapatinib, axitinib, pazopanib, sunitinib, and sorafenib.

In the present invention, the immuno-anticancer agent includes a CTLA-4 antibody such as ipilimumab, PD-1 antibodies such as pembrolizumab and nivolumab, a PD-L1 antibody such as atezolizumab, or an immune checkpoint inhibitor such as an IDO inhibitor, but is not limited thereto.

In the present invention, the hormonal anticancer agent includes, but is not limited to, male hormone inhibitors such as bicalutamide and enzalutamide, and female hormone inhibitors such as tamoxifen, anastrozole and letrozole.

In the present invention, the cell therapy agent means a therapeutic agent using live immune cells as an active ingredient, and includes, but is not limited to, a cytotoxic T cell therapy agent, a CAR-T cell therapy agent, a CAR-NK cell therapy agent, and the like.

The *L. plantarum* IMB19 strain of the present invention exhibits various immune-enhancing activities and/or anti-tumor activities of i) inducing T cell differentiation in an inflammatory direction, such as induction of helper T cells and inhibition of Treg cell differentiation, ii) stimulating CD8+ T cells, enhancing the activity thereof and improving intratumoral infiltration, iii) inhibiting Treg cell activity, iv) increasing intratumoral macrophage infiltration, and v) activating macrophages into inflammatory cells and reprogramming M2 to M1 macrophages. In the present invention, preferably, the anti-cancer agent may be an immuno-anticancer agent.

In the present invention, more preferably, the immuno-anticancer agent may be an immune checkpoint inhibitor.

In the present invention, the immune checkpoint inhibitor is an agent that blocks signaling by a receptor involved in an immune checkpoint or a ligand thereof, and inhibits an immune checkpoint protein selected from the group consisting of PD-1/PD-L1, CTLA-4, IDO, B7-1, and B7-2.

In the present invention, the immune checkpoint inhibitor may be an antibody that specifically binds to the immune checkpoint protein. In the present invention, the antibody that specifically binds to the immune checkpoint protein is, for example, a PD-1 inhibitor such as pembrolizumab, nivolumab, or cemiplimab, a PD-L1 inhibitor such as atezolizumab, avelumab or durvalumab, and a CTLA-4 blocker such as atezolimumab, but is not limited thereto.

In one embodiment of the present invention, the animal tumor model fed the strain exhibits significant immune-enhancing effects and antitumor activity by various mechanisms such as stimulation of CD8+ T cells, differentiation in macrophages, induction of phenotypes and iron sequestration, suppression of Tregs, enhancement of tumor infiltration of immune cells, and regulation of TCR repertoire of CD8+ T cells.

In addition, in another embodiment of the present invention, it was found that the growth of tumors was remarkably suppressed by CPS treatment.

As used herein, the term *"*tumor*"* includes all phenomena in which cells proliferate autonomously and excessively after escaping from the biological regulatory mechanisms, or neoplasms or hyperplasias generated thereby. The tumor includes, for example, benign, premalignant, and malignant tumors, and more specifically, examples thereof include histiocytoma, glioma, astrocytoma, osteoma, and various cancers, such as lung cancer, small cell lung cancer, stomach cancer, gastrointestinal cancer, bowel cancer, colon cancer, rectal cancer, pancreatic cancer, breast cancer, skin cancer, ovarian cancer, prostate cancer, testicular cancer, liver cancer, kidney cancer, bladder cancer, pancreatic cancer, brain cancer, sarcoma, osteosarcoma, melanoma, lymphoma (Hodgkin's lymphoma, FL, MCL, MZBL , CLL, T-ALL, AML, ALL, etc.), blood cancer, leukemia, psoriasis, bone disease, fibroproliferative disorder, atherosclerosis, and the like. The tumor is preferably melanoma, breast cancer, kidney cancer, lung cancer, bladder cancer, or rectal cancer, but is not limited thereto.

As used herein, the term *"*prevention*"* refers to any action that suppresses or delays the onset of diseases by administration of the composition according to the present invention.

As used herein, the term *"*treatment*"* means any action that can ameliorate or beneficially alter the symptoms of diseases by administration of the composition according to the present invention.

The composition of the present invention exhibits prophylactic or therapeutic and anti-inflammatory effects for various diseases through the immune enhancing effect and/or anti-tumor effect of the active ingredient.

The composition of the present invention may be a composition for co-administration with an anticancer agent.

In the present invention, the *L. plantarum* IMB19 strain and the anticancer agent may be mixed and contained in the composition as a single formulation for simultaneous administration, but is not limited thereto. The composition containing the *L*. *plantarum* IMB19 strain and the composition containing the anticancer agent are prepared as separate formulations.

In the present invention, the *L. plantarum* IMB19 strain and the anticancer agent may be administered through the same or different routes of administration. For example, the *L. plantarum* IMB19 strain is a formulation suitable for oral administration and the anticancer agent may be prepared and administered as an injection formulation, but this is provided as an example for illustration and is not limited thereto.

In the present invention, the composition containing the *L. plantarum* IMB19 strain is preferably prepared as an oral formulation, but is not limited thereto. In the present invention, the composition containing the *L. plantarum* IMB19 strain may be used in the form of a composition suitable for administration to humans or animals.

The *L. plantarum* IMB19 strain, which is an active ingredient of the composition of the present invention, may be provided as a composition in the form of a lyophilizate, capsule, culture suspension, fermentation broth, dry powder or granule when prepared as an oral formulation.

When a parenteral formulation of the *L. plantarum* IMB19 strain, which is the active ingredient of the composition of the present invention, is prepared, it may contain a sterile aqueous solution, a non-aqueous solvent, a suspension, an emulsion, a lyophilizate, and a suppository. Examples of the non-aqueous solvent and suspension include propylene glycol, polyethylene glycol, vegetable oil such as olive oil, injectable esters such as ethyl oleate, and the like. Examples of the suppository base include Witepsol, macrogol, Tween 60, cacao butter, laurin butter, glycerogelatin and the like. Suitable formulations known in the art may be prepared according to the method disclosed in the document (Remington's Pharmaceutical Science (latest edition), Mack Publishing Company, Easton PA).

In the present invention, the composition may further contain suitable carriers, excipients and diluents commonly used in addition to the *L. plantarum* IMB19 strain.

The carrier, excipient or diluent that may be contained in the composition includes lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. The formulation of the composition may be prepared using a commonly used diluent or excipient such as a filler, extender, binder, wetting agent, disintegrant, or surfactant.

The composition according to the present invention can be formulated and used in various forms according to a conventional method. Suitable formulations include oral formulations such as tablets, pills, powders, granules, dragées, hard or soft capsules, solutions, suspensions, emulsions, injections and aerosols, external preparations, suppositories, sterile injectable solutions, and the like, but are not limited thereto.

The composition according to the present invention can be prepared into a suitable formulation using a pharmaceutically inactive organic or inorganic carrier. That is, when the formulation is a tablet, a coated tablet, a dragée or a hard capsule, it may contain lactose, sucrose, starch or a derivative thereof, talc, calcium carbonate, gelatin, stearic acid, or a salt thereof. In addition, when the formulation is a soft capsule, it may contain a vegetable oil, wax, fat, or semi-solid or liquid polyol. In addition, when the formulation is in the form of a solution or syrup, it may contain water, polyol, glycerol, vegetable oil, or the like.

The composition according to the present invention may further contain a preservative, a stabilizer, a wetting agent, an emulsifier, a solubilizing agent, a flavoring agent, a colorant, an osmotic pressure regulator, an antioxidant or the like, in addition to the above carrier.

The composition of the present invention may be prepared as an enteric coating formulation using a method known in the art so that *L. plantarum* IMB19 strain, the microorganism, which is an active ingredient, can be rapidly released into the intestines after passing through the stomach and reaching the small intestine.

In addition, the composition of the present invention may be prepared as a capsule using a conventional encapsulation method. For example, pellets containing the lyophilized microorganisms of the present invention are prepared using standard carriers and then charged in hard gelatin capsules. Alternatively, a suspension or dispersion may be prepared using the microorganism of the present invention along with any suitable pharmaceutical carrier, such as aqueous gum, cellulose, silicate or oil, and then may be charged into soft gelatin capsules.

In the present invention, the composition may be provided as an enteric coated preparation, in particular, as a unit oral formulation. As used herein, the term *"*enteric coating*"* includes all types of pharmaceutically acceptable coatings that are not degraded by gastric acid, but are sufficiently degraded in the small intestine to release the active ingredient into the small intestine. The material for the enteric coating may be appropriately selected from known polymer materials and suitable polymer materials are described in a number of known publications (L. Lachman et al., The Theory and Practice of Industrial Pharmacy, 3rd ed., 1986, pp. 365-373; H. Sucker et al., Pharmazeutische Technologie, Thieme, 1991, pp. 355-359; Hagers Handbuch der pharmazeutischen Praxis, 4th ed., Vol. 7, pp. 739-742, and 766-778, (Springer Verlag, 1971); and Remington's Pharmaceutical Sciences, 13th ed., pp. 1689-1691 (Mack Publ., Co., 1970)) and include, but are not limited to, cellulose ester derivatives, cellulose ethers, methyl acrylate copolymers of acrylic resins, and copolymers of maleic acid and phthalic acid derivatives.

The composition according to the present invention is administered in an effective dose. For example, when the composition is a food composition or a pharmaceutical composition, it may be administered in a foodologically effective dose or a pharmaceutically effective amount, respectively. In the present invention, *"*effective dose*"*, *"*foodologically effective dose*"* and *"*pharmaceutically effective amount*"* mean an amount sufficient to treat a disease with a reasonable benefit/risk ratio applicable to prevention or treatment, which is the object of the present invention and the effective dosage level may be determined depending on a variety of factors including the type of the disease of the patient, the severity of the disease, the activity of the drug, the sensitivity of the patient to the drug, the administration time, the administration route, the excretion rate, the treatment period, drugs used concurrently therewith, and other factors well-known in the pharmaceutical field.

The composition of the present invention may be administered as a single therapeutic agent or in combination with other therapeutic agents, may be administered sequentially or simultaneously with a conventional therapeutic agent, and may be administered in single or multiple doses. Taking into consideration these factors, it is important to administer the minimum amount sufficient to achieve maximum efficacy without side effects and the amount can be easily determined by those skilled in the art.

For example, in the present invention, the *L. plantarum* IMB19 strain is administered at a dose of about 1×10¹ CFU to about 1×10²⁰ CFU, preferably about 1×10⁵ CFU to about 1×10¹⁵ CFU. In one embodiment of the present invention, the *L. plantarum* IMB19 strain is administered in a dose of about 1×10⁹ CFU, but is not limited thereto.

In the present invention, the anticancer agent administered in combination with the composition of the present invention may be administered in a pharmaceutically effective dose known in the art depending on the anticancer agent. In one embodiment of the present invention, the PD-L1 antibody concomitantly administered with an anticancer agent was administered at a dose of 100 µg, but is not limited thereto.

In the present invention, the administration may be performed through various routes. For example, the mode of administration may be subcutaneous, intravenous, intramuscular, intrauterine, intrathecal or intracerebrovascular injection. The composition of the present invention is determined depending on the type of drug as an active ingredient, along with various related factors such as the disease to be treated, the route of administration, the age, gender and weight of the patient, and the severity of the disease.

The administration method of the composition according to the present invention can be easily selected depending on the formulation and can be administered orally or parenterally. The dosage may vary depending on the age, gender, and weight of the patient, severity of symptoms, and route of administration. The composition of the present invention may be administered in combination with other therapy or drug. When used for the prevention or treatment of tumors, preferably, the other therapy or drug may be an immunotherapy or an immune cell therapy agent, but is not limited thereto, and may be used in various combinations by the prescription of clinicians.

As used herein, the term *"*co-administration*"* means that two or more types of active ingredients are administered simultaneously or sequentially, or administered at a specific interval to obtain an improved effect when each active ingredient is independently administered, based on the action of two or more types of active ingredients.

In the present invention, the co-administration may be performed by administration of the *L. plantarum* IMB19 strain in combination with an anticancer agent, and may be further used in combination with a composition containing other active ingredient or other therapy.

In the present invention, the active ingredients are co-administered through independent routes. Each active ingredient may be independently administered in an appropriate dosage using a suitable administration regimen by those skilled in the art. For example, as in one embodiment of the present invention, preferably, the *L. plantarum* IMB19 strain is administered orally and the anticancer agent is administered through intravenous injection, but the present invention is not limited thereto.

In the present invention, the co-administration may be performed by simultaneously administering the *L. plantarum* IMB19 strain and the anticancer agent.

In the present invention, the co-administration may be performed by sequentially administering the *L. plantarum* IMB19 strain and the anticancer agent. For example, the anticancer agent is administered after administration of the *L. plantarum* IMB19 strain, or the *L. plantarum* IMB19 strain is administered after the administration of the anticancer agent.

In the present invention, when the *L. plantarum* IMB19 strain and the anticancer agent are sequentially administered, they are administered at a regular time interval, which includes, but is not limited to, a 1-minute interval, a 5-minute interval, a 10-minute interval, a 20-minute interval, a 30-minute interval, a 1-hour interval, a 1-day interval, a several-day interval, or a several-week interval, and are administered sequentially at appropriate intervals by those skilled in the art.

In the present invention, the administration of the *L*. *plantarum* IMB19 strain and the anticancer agent may be repeatedly performed one or more times. When the active ingredients of the present invention, the *L. plantarum* IMB19 strain and the anticancer agent are repeatedly administered, the administration interval can be easily adjusted by those skilled in the art according to the condition of the subject to which they are administered and the levels of the desired effect. For example, the *L. plantarum* IMB19 strain and the anticancer agent may be administered through an interval of a 1-hour interval, a 6-hour interval, an 8-hour interval, a 12-hour interval, a 1-day interval, a 2-day interval, a 1-week interval, a 2-week interval, or a 1-month interval, but is not limited thereto.

The composition of the present invention may be prepared as a pharmaceutical composition or a food composition.

In the present invention, the composition containing the *L. plantarum* IMB19 strain may be administered in the form of a composition, for example, a pharmaceutical composition or a food composition, independent of a formulation of the anticancer agent administered in combination therewith. Preferably, the pharmaceutical composition containing the *L. plantarum* IMB19 strain and the pharmaceutical composition containing the anticancer agent are each administered at an effective dose, or a food composition containing the *L. plantarum* IMB19 strain and a pharmaceutical composition containing the anticancer agent are administered at an effective dose, but the present invention is not limited thereto.

As used herein, the term *"*food composition*"* is used in a broad sense to encompass substances containing nutrients, and examples of the food composition include meat, sausage, bread, chocolate, candy, snacks, confectionery, pizza, ramen, other noodles, gum, dairy products including ice cream, various soups, beverages, tea, drinks, alcoholic beverages, vitamin complexes, prebiotics, probiotics, postbiotics, health supplement food, health functional food, health food and the like. The term *"*food composition*"* is used in an ordinary sense to encompass *"*food*"* and *"*food additive*"* or *"*composition for food additive*"* added to food.

In the present invention, when the food is provided as feed for animals other than humans, it may be used interchangeably in substantially the same sense as *"*feed*"*. Therefore, in the present invention, the food composition may mean a feed composition or feed additive (feed additive composition).

As used herein, the term *"*functional food*"* has the same meaning as the term *"*food for special health use (FoSHU)*"* and refers to a food having strong medical and pharmaceutical effects that has been processed to efficiently provide a bioregulatory function as well as a nutrition supply function. Here, the term *"*functional*"* means obtaining beneficial effects for health purposes, such as controlling nutrients or exhibiting physiological effects with regard to the structures and functions of the human body. The food of the present invention may be prepared by a method commonly used in the art, and the preparation may be performed using raw materials and ingredients commonly added in the art. In addition, the food may also be prepared into any formulation recognized as a food without limitation, and the health functional food according to the present invention may be in the form of a powder, granule, tablet, capsule, or beverage.

The term *"*health food*"* refers to a food having an active health maintenance or promotion effect beyond that of a general food, and the term "health supplement food" refers to a food ingested for the purpose of health supplement. In some cases, the terms "health functional food", "health food", and "health supplement food" are used interchangeably.

The food composition may further contain a physiologically acceptable carrier, and there is no particular limitation as to the kind of carrier, and any carrier commonly used in the art may be used.

In addition, the composition may contain additional ingredients that are commonly used in food compositions to improve smell, taste, visual quality (appearance), and the like. For example, the composition may contain vitamins A, C, D, E, B1, B2, B6 and B12, niacin, biotin, folate, pantothenic acid, and the like. In addition, the composition may contain minerals such as zinc (Zn), iron (Fe), calcium (Ca), chromium (Cr), magnesium (Mg), manganese (Mn), copper (Cu), and chromium (Cr). In addition, the composition may contain amino acids such as lysine, tryptophan, cysteine, and valine.

In addition, the composition may contain food additives such as preservatives (potassium sorbate, sodium benzoate, salicylic acid, sodium dehydroacetate, etc.), disinfectants (bleached powder and highly bleached powder, sodium hypochlorite, etc.), antioxidants (butylhydroxyanisole (BHA), butylhydroxytoluene (BHT), etc.), colorants (tar color, etc.), color-developing agents (sodium nitrite, sodium nitrite, etc.), bleaching agents (sodium sulfite), seasonings (MSG, etc.), sweeteners (dulcin, cyclamate, saccharin, sodium, etc.), flavorings (vanillin, lactones, etc.), expanding agents (alum, D-potassium hydrogen tartrate, etc.), reinforcing agents, emulsifiers, thickeners, coating agents, gum base agents, antifoaming agents, solvents, and enhancers. The additive may be selected according to the type of food and used in an appropriate amount.

In addition to the *L. plantarum* IMB19 strain of the present invention, the composition may further contain a foodologically acceptable food supplement additive, may be used in combination with other foods or food ingredients, and may be appropriately used according to a conventional method. The amount of the active ingredient that is mixed may be appropriately determined according to the purpose of use (prevention, health or therapeutic treatment).

In another aspect, the present invention is directed to a pharmaceutical composition for preventing or treating tumors containing a *Lactobacillus plantarum* IMB19 strain KCTC 14337BP and an anticancer agent.

In another aspect, the present invention is directed to a method for preventing or treating tumors including co-administering the *Lactobacillus plantarum* IMB19 strain KCTC14337BP and an anticancer agent to a subject.

In another aspect, the present invention is directed to the use of co-administration of the *Lactobacillus plantarum* IMB19 strain KCTC14337BP with an anticancer agent for the prevention, amelioration or treatment of tumors.

In another aspect, the present invention is directed to the use of the *Lactobacillus plantarum* IMB19 strain KCTC14337BP and an anticancer agent for the preparation of a composition for co-administration.

In one embodiment of the present invention, it was found that capsular polysaccharides are active ingredients exhibiting the immune-enhancing activity of the *L. plantarum* IMB19 of the present invention.

In addition, in another embodiment of the present invention, like the *L. plantarum* IMB19 strain, the isolated capsular polysaccharide exhibits excellent immune-enhancing activities and/or anti-tumor activities through various mechanisms of i) inducing T cell differentiation in an inflammatory direction, such as induction of helper T cells and inhibition of Treg cell differentiation, ii) stimulating CD8+ T cells, enhancing the activity thereof and improving intratumoral infiltration, iii) inhibiting Treg cell activity, iv) increasing intratumoral macrophage infiltration, v) activating macrophages into inflammatory cells and reprogramming M2 to M1 macrophages, vi) altering gene profiles in macrophages and iron sequestration, and/or vii) modulating the TCR repertoire of CD8+ T cells.

In another embodiment of the present invention, the capsular polysaccharide was structurally analyzed by NMR.

Therefore, in another aspect, the present invention is directed to a composition for preventing or treating tumors containing capsular polysaccharides (CPS) derived from a *Lactobacillus plantarum* IMB19 strain KCTC 14337BP having an immune-enhancing activity, the pharmaceutical composition being administered in combination with an anticancer agent.

In the present invention, the capsular polysaccharide may include a polysaccharide of the following Formula (I):

[Formula I] -[-D-B-I-F-G-C-E-H-A-]ₙ-

wherein A and D are galactose;
B, C, E, G, and H are rhamnose;
F is N-acetylglucosamine;
I is glucose; and
n is an integer of 1 or more.

In the present invention, the polysaccharide of Formula I may have a polymer (n=1 or more) structure of repeating units represented by [-D-B-I-F-G-C-E-H-A-] in Formula I.

In the present invention, when n in Formula I is 2 or more, the repeating units may be linked without limitation by various methods other than a direct covalent bond. For example, the repeating units may be linked by a chemical bond such as a glycosidic linkage or a phosphodiester linkage, or may be linked via a linker.

In the present invention, when n in Formula I is 2 or more, the repeating units ([-D-B-I-F-G-C-E-H-A-]) are linked via a glycosidic linkage (-O-) between A and D.

In the present invention, when n in Formula I is 2 or more, the repeating units ([-D-B-I-F-G-C-E-H-A-]) may be linked by a phosphodiester linkage between A and D.

In the present invention, when n in Formula I is 2 or more, carbon 1 of A of one repeating unit and carbon 6 of D of another repeating unit may be linked by a phosphodiester linkage.

In the present invention, at least one of A or D of Formula I may be phosphorylated. Preferably, the hydroxyl group of carbon 1 in A is phosphorylated and the hydroxyl group of carbon 6 in D is phosphorylated.

In the present invention, the galactose includes naturally derived typical galactose or a derivative thereof. For example, in the present invention, the galactose may be an isomer, specifically, an α-type (α configuration), β-type (β configuration), D-type (D configuration), or L-type (L configuration) isomer, preferably α-galactose, more preferably, α-D-galactose, but is not limited thereto.

In the present invention, the rhamnose includes naturally derived typical rhamnose or a derivative thereof. For example, in the present invention, the rhamnose may be an isomer, specifically, an α-type (α configuration), β-type (β configuration), D-type (D configuration), or L- (L configuration) isomer, preferably α-rhamnose, more preferably, α-D-rhamnose, but is not limited thereto.

In the present invention, the N-acetylglucosamine includes N-acetylglucosamine and derivatives thereof.

In the present invention, the glucose includes all general glucose or derivatives thereof. For example, in the present invention, the glucose may be an isomer, specially, an α-, β-, D- or L-isomer, preferably -β glucose having a β-type configuration, more preferably D-glucose, but is not limited thereto.

In the present invention, D and B (D-B) and B and I (B-I) may be linked by an α-1,3-glycosidic linkage.

In the present invention, the I and F (I-F) may be linked by a β-1,6-glycosidic linkage.

In the present invention, the F and G (F-G), G and C (G-C), C and E (C-E), and E and H (E-H) are linked by α-1,2-glycosidic linkages.

In the present invention, the H and A (H-A) may be linked by an α-1,6-glycosidic linkage.

Here, the glycosidic linkage is expressed as α or β depending on the bonding orientation and the following numbers refer to the number of carbon linked by the glycosidic linkage (-O-) in each of two monosaccharides. For example, in the *"*I and F (I-F) are linked by a β-1,6-glycosidic linkage", carbon 1 of I is linked to carbon 6 of F via a β-configuration glycosidic linkage based on I.

In the present invention, the capsular polysaccharide may include a polysaccharide of Formula I where n is an integer of 1 or more and may include a plurality of polysaccharides of Formula I polymerized at an integer of a variable n. Preferably, in the polysaccharide of Formula I, n is 1 to 10.

In one embodiment of the present invention, the average degree of polymerization of the polysaccharide of Formula I contained in the capsular polysaccharide was about 4, and the average molecular weight was found to be about 6.0 kDa. Each repeating unit ([-D-B-I-F-G-C-E-H-A-]) was found to have a molecular weight (MW) of about 1.5 kDa.

In the present invention, the "average of n" of the polysaccharide of Formula I included in the capsular polysaccharide may be preferably 1 to 10, and most preferably about 4.

In the present invention, the polysaccharide of Formula I included in the capsular polysaccharide may have an average molecular weight of 1.5 to 15 kDa, preferably about 6.0 kDa.

In one embodiment of the present invention, the polysaccharide of Formula I included in the capsular polysaccharide is capsular polysaccharide (CPS) derived from the *L. plantarum* IMB19 strain by ion exchange chromatography using about 100 mM NaCl as an eluent. The separation method are described in detail in Examples, but is not limited thereto, and may be obtained through various conventionally known purification methods based on the polysaccharide structure and characteristics of Formula I described in the present invention.

In the present invention, the capsular polysaccharide may further contain teichoic acid.

As can be seen from one embodiment of the present invention, in the present invention, the capsular polysaccharide may further contain two or more teichoic acids.

In the present invention, the teichoic acid may be Gro-type or Rbo-type, and when the capsular polysaccharide further contains two or more teichoic acids, the teichoic acids may be contained as a single type or a mixture of the two different types.

In the present invention, the capsular polysaccharide may further include other polysaccharides or biomolecules such as lipids in addition to the polysaccharide of Formula I and/or teichoic acid.

In another aspect, the present invention is directed to a pharmaceutical composition for preventing or treating tumors containing capsular polysaccharides (CPS) derived from *Lactobacillus plantarum* IMB19 strain KCTC 14337BP and an anticancer agent.

In another aspect, the present invention is directed to a method for preventing or treating tumors including co-administering the capsular polysaccharides (CPS) derived from *Lactobacillus plantarum* IMB19 strain KCTC 14337BP and an anticancer agent to a subject.

In another aspect, the present invention is directed to the use of co-administration of the capsular polysaccharides (CPS) derived from the *Lactobacillus plantarum* IMB19 strain KCTC14337BP with an anticancer agent for the prevention, amelioration or treatment of tumors.

In another aspect, the present invention is directed to the use of the capsular polysaccharides (CPS) derived from the *Lactobacillus plantarum* IMB19 strain KCTC14337BP and the anticancer agent for the preparation of a composition for co-administration.

The method of producing capsular polysaccharides according to the present invention and an example of the step of obtaining an effective polysaccharide fraction having immune-enhancing activity is described in detail in Examples of the present invention, but is not limited thereto.

In the examples of the present invention, capsular polysaccharides (CPS) derived from the *L. plantarum* IMB19 strain were fractionated by ion exchange chromatography using various concentrations of NaCl eluent, and the structure of the polysaccharides contained in each fraction was analyzed by NMR.

In another embodiment of the present invention, among the various fractions of capsular polysaccharides (CPS) derived from the *L. plantarum* IMB19 strain, only CPS-100 purified using 100 mM NaCl produced significant levels of IFN-γTNF-α, IL-6 and IL-12, and negligible levels of IL-10, IL-17, and IL1-β, while other fractions (such as CPS-400 containing teichoic acid) did not produce significant cytokines, which indicates that the effective molecule exhibiting the immune-enhancing activity of the *L*. *plantarum* IMB19 strain was a polysaccharide (Formula I) having a polymer structure of specific repeating units included in CPS-100.

In another aspect, the present invention is directed to a composition for preventing or treating tumors containing a polysaccharide represented by the following Formula (I), the composition being administered in combination with an anticancer agent.

[Formula I] -[-D-B-I-F-G-C-E-H-A-]ₙ-

wherein
A and D are galactose;
B, C, E, G and H are rhamnose;
F is N-acetylglucosamine;
I is glucose; and
n is an integer from 1 to 10.

In the present invention, the polysaccharide of Formula I may have a polymer (n≥1) structure of repeating units represented by [-D-B-I-F-G-C-E-H-A-] in Formula I.

In the present invention, when n in Formula I is 2 or more, the repeating units may be linked without limitation by various methods other than a direct covalent bond. For example, the repeating units may be linked by a chemical bond such as a glycosidic linkage or a phosphodiester linkage, or may be linked via a linker.

In the present invention, when n in Formula I is 2 or more, the repeating units ([-D-B-I-F-G-C-E-H-A-]) are linked via a glycosidic linkage (-O-) between A and D.

In the present invention, when n in Formula I is 2 or more, the repeating units ([-D-B-I-F-G-C-E-H-A-]) may be linked by a phosphodiester linkage between A and D.

In the present invention, when n in Formula I is 2 or more, carbon 1 of A of one repeating unit and carbon 6 of D of another repeating unit may be linked by a phosphodiester linkage.

In the present invention, at least one of A or D of Formula I may be phosphorylated. Preferably, the hydroxyl group of carbon 1 in A is phosphorylated and the hydroxyl group of carbon 6 in D is phosphorylated.

In the present invention, the galactose includes naturally derived typical galactose or a derivative thereof. For example, in the present invention, the galactose may be an isomer, specifically, an α-type (α configuration), β-type (β configuration), D-type (D configuration), or L-type (L configuration) isomer, preferably α-galactose, more preferably, α-D-galactose, but is not limited thereto.

In the present invention, the rhamnose includes naturally derived typical rhamnose or a derivative thereof. For example, in the present invention, the rhamnose may be, as an isomer, α-type (α configuration), β-type (β configuration), D-type (D configuration), or L-type (L configuration), preferably α-rhamnose, more preferably, α-D-rhamnose, but is not limited thereto.

In the present invention, the N-acetylglucosamine includes naturally derived typical N-acetylglucosamine and derivatives thereof.

In the present invention, the glucose includes all general glucose or derivatives thereof. For example, in the present invention, the glucose may be an isomer, specifically, α-type or β-type, D-type or L-type, preferably β glucose having a β-configuration, more preferably D-glucose, but is not limited thereto.

As used herein, the term "derivative" refers to a compound that has a sufficiently similar structure to the compound disclosed herein and exhibits the same or similar activities and uses as the claimed compound based on such similarity, or a compound, as a precursor, that has a structure derived from the structure of a parent compound (e.g., a compound described herein, a polysaccharide of Formula I) that is expected to exhibit the same or similar activities and uses as the claimed compound. For example, the derivative includes, but is not limited to, salts, isomers, esters, amides, and esters of the parent compound or salts of amides, N-oxides, and the like.

In the present invention, D and B (D-B) and B and I (B-I) in Formula I may be linked by an α-1,3-glycosidic linkage.

In the present invention, the I and F (I-F) may be linked by a β-1,6-glycosidic linkage.

In the present invention, the F and G (F-G), G and C (G-C), C and E (C-E), and E and H (E-H) are linked by α-1,2-glycosidic linkages.

In the present invention, the H and A (H-A) in Formula I may be linked by an α-1,6-glycosidic linkage.

Here, the linkage is expressed as α or β depending on the bonding orientation and the following numbers refer to the number of carbons linked by the glycosidic linkage (-0-) in each of the two monosaccharides.

In the present invention, n in Formula I may be an integer of 1 or more, preferably an integer of 1 to 10, more preferably n is 4.

In the present invention, the polysaccharide may have a structure of the following Formula II: 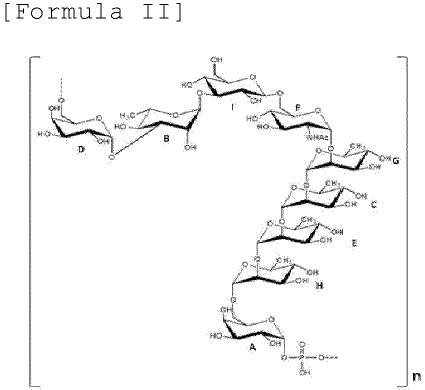 wherein n is an integer of 1 or more.

In the present invention, n in Formula II is preferably an integer of 1 to 10, more preferably n is 4.

In one embodiment of the present invention, the average degree of polymerization of the polysaccharide of Formula I contained in CPS-100, the active fraction of the capsular polysaccharide, was about 4, and the average molecular weight was found to be about 6.0 kDa. Each repeating unit ([-D-B-I-F-G-C-EH-A-]) was found to have a molecular weight (MW) of about 1.5 kDa.

The polysaccharide of Formula I included as an active ingredient in the composition of the present invention may contain two or more polysaccharides having different degrees of polymerization (n).

In the present invention, the "average of n" of the polysaccharide of Formula I contained in the composition may be preferably 1 to 10, and most preferably about 4.

In the present invention, the polysaccharide of Formula I may have an average molecular weight of at least about 1.5 kDa, preferably about 1.5 kDa to about 15 kDa, more preferably about 4 kDa, depending on the degree of polymerization, degree of phosphorylation, or the like.

In the present invention, the polysaccharide may be derived from the *Lactobacillus plantarum* IMB19 strain KCTC14337BP.

In the present invention, the polysaccharide of Formula I may have immunity enhancement and/or antitumor activity. As a more specific example, the *L. plantarum* IMB19 strain exhibits various immune-enhancing activities and/or anti-tumor activities through various mechanisms, including, but are not limited to, i) inducing T cell differentiation in an inflammatory direction, such as induction of helper T cells and inhibition of Treg cell differentiation, ii) stimulating CD8+ T cells, enhancing the activity thereof and improving intratumoral infiltration, iii) inhibiting Treg cell activity, iv) increasing intratumoral macrophage infiltration, v) activating macrophages into inflammatory cells and reprogramming M2 to M1 macrophages, vi) altering gene profiles in macrophages and iron sequestration, and/or vii) modulating the TCR repertoire of CD8+ T cells.

In the present invention, the polysaccharide of Formula I may inhibit tumor growth.

In the present invention, those skilled in the art can produce/isolate the polysaccharide using the strain of the present invention, and induce or synthesize the polysaccharide of the present invention through other chemical or biological methods.

Furthermore, it is apparent that modification of the structure of CPS for clinical formulation (e.g., solubility) or improvement of pharmacokinetic and/or pharmacodynamic properties of the polysaccharide of the present invention can be performed. For example, i) addition of one or more functional groups, ii) modification of the carbon chains, iii) addition of one or more hydrogen or hydroxyl groups, iv) modification of terminal groups (e.g. addition of signal molecules such as dyes), and v) binding with known other sugar molecules (e.g., formulations for oral or systemic delivery, and the like) may be performed, but is not limited thereto.

Therefore, the polysaccharide of the present invention is not limited to the polysaccharide of Formula I or Formula II, and includes all variants, derivatives, analogues, and the like of Formula I or Formula II as long as they have immune-stimulating and immune-enhancing effects.

Therefore, in another aspect, the present invention is directed to a composition for modulating containing the polysaccharide of Formula I as an active ingredient.

In another aspect, the present invention is directed to a method of modulating immunity including administering the polysaccharide to a subject.

In another aspect, the present invention is directed to the use of the polysaccharide for immunomodulation.

In another aspect, the present invention is directed to the use of a polysaccharide for the preparation of a composition for modulating immunity.

Hereinafter, unless there is a specific definition of the term, it will be understood the same as the meaning understood by those skilled in the art or the meaning defined in another aspect of the present invention.

In one embodiment of the present invention, the half maximal effective concentration (EC50) of the polysaccharide was found to be 3.16 µM. Therefore, preferably, the pharmaceutical composition of the present invention may contain at least 3.16 µM or more of polysaccharides.

In the present invention, the polysaccharide and the anticancer agent may be mixed and contained in the composition as a single formulation for simultaneous administration, but is not limited thereto. The composition containing the polysaccharide and the composition including the anticancer agent are prepared as separate formulations.

In the present invention, unless otherwise mentioned, the formulation, administration regimen, dosage, co-administration, and the like of the composition containing the polysaccharide may be characterized as having substantially the same characteristics as described in terms of the composition for preventing or treating tumors containing the *Lactobacillus plantarum* IMB19 strain KCTC14337BP, the composition being administered in combination with an anticancer agent.

In the present invention, the co-administration of the polysaccharide and the anticancer agent may be performed by simultaneously or sequentially administering the polysaccharide and the anticancer agent. For example, the anticancer agent is administered after administration of the polysaccharide, or the polysaccharide is administered after the administration of the anticancer agent.

In the present invention, when the polysaccharide and an anticancer agent are sequentially administered, they are administered at a regular time interval, which includes, but is not limited to, a 1-minute interval, a 5-minute interval, a 10-minute interval, a 20-minute interval, a 30-minute interval, a 1-hour interval, a 1-day interval, a several-day interval, or a several-week interval, and are administered sequentially at appropriate intervals by those skilled in the art.

In the present invention, the administration of the polysaccharide and the anticancer agent may be repeatedly performed one or more times. When the active ingredients of the present invention, the polysaccharide and the anticancer agent are repeatedly administered, the administration interval can be easily adjusted by those skilled in the art according to the condition of the subject to which they are administered and the levels of the desired effect. For example, the polysaccharide and the anticancer agent may be administered through an interval of a 1-hour interval, a 6-hour interval, an 8-hour interval, a 12-hour interval, a 1-day interval, a 2-day interval, a 1-week interval, a 2-week interval, or a 1-month interval, but is not limited thereto.

Therefore, in another aspect, the present invention is directed to a pharmaceutical composition for preventing or treating tumors containing the polysaccharide of Formula I as an active ingredient.

In another aspect, the present invention relates to a method for preventing or treating tumors including coadministering the polysaccharide and the anticancer agent to a subject.

In another aspect, the present invention is directed to the use of the polysaccharide of co-administration with the anticancer drug for the prevention, amelioration or treatment of tumors.

In another aspect, the present invention is directed to the use of the polysaccharide of Formula I for the preparation of a composition for co-administration with the anticancer drug for the prevention, amelioration or treatment of tumors.

As used herein, the term "tumor" includes all phenomena in which cells proliferate autonomously and excessively after escaping from the biological regulatory mechanisms, or neoplasms or hyperplasias generated thereby. The tumor includes, for example, benign, premalignant, and malignant tumors, and more specifically, examples thereof include histiocytoma, glioma, astrocytoma, osteoma, and various cancers, such as lung cancer, small cell lung cancer, stomach cancer, gastrointestinal cancer, bowel cancer, colon cancer, rectal cancer, pancreatic cancer, breast cancer, skin cancer, ovarian cancer, prostate cancer, testicular cancer, liver cancer, kidney cancer, bladder cancer, pancreatic cancer, brain cancer, sarcoma, osteosarcoma, melanoma, lymphoma (Hodgkin's lymphoma, FL, MCL, MZBL , CLL, T-ALL, AML, ALL, etc.), blood cancer, leukemia, psoriasis, bone disease, fibroproliferative disorder, atherosclerosis, and the like, but is not limited thereto.

### Example

Hereinafter, the present invention will be described in more detail with reference to examples. However, it will be obvious to those skilled in the art that these examples are provided only for illustration of the present invention, and should not be construed as limiting the scope of the present invention.

### Example 1: Materials and Methods

### 1. Bacterial culture and identification

Kimchi was homogenized and the suspension was collected. Then, the suspension was serially diluted, streaked on MRS broth and agar, and cultured at 37°C for 48 hours to isolate colonies and conduct further culture for various assays.

The *Lactobacillus plantarum* IMB19 strain was generally cultured in MRS broth at 37°C for 24 to 36 hours. For transmission electron microscopy (TEM), the bacteria was cultured, streaked on MRS-1.5% agar (Neogen Corp., USA) and incubated for 24 to 30 hours. Bacterial colonies were drop-cast onto 2,000 mesh graphene-coated copper grids. TEM imaging was performed at 80 kV using a JEOL1220 and Hitachi HT7700.

For identification, the cell morphology of the selected isolates was observed under a microscope, genetic characterization was performed using genomic DNA, and 16s rRNA sequencing was performed by Macrogen (Korea).

The 16S rRNA gene was amplified by direct PCR using the universal primers of forward (27F primer), 5'-AGAGTTTGATCMTGGCTCAG-3' and reverse (1492R primer), 5'-TACGGYTACCTTGTTACGACTT-3' primers.

### 2. Primary cell-based tests

### 2-1. In vitro splenocyte stimulation

All animal experiments and procedures were performed in accordance with the ethical regulations and approval of the Animal Care and Use Committee of Pohang University of Science and Technology. C57BL/6 mice were reared and bred in a pathogen-free animal barrier facility and the age of mice used was 6-8 weeks old. Spleens were harvested and gently triturated to release splenocytes. The cell suspension was RBC-lysed using ammonium chloride buffer and resuspended in complete RPMI medium (Welgene, S. Korea) containing 10% FBS (Hy-Clone, Australia). Cells were plated at a density of 200 k/well in 200 µL of media/well containing 10 ng/mL of anti-CD3 (Bio-Xcell, USA) and 2.5 ng/mL of GM-CSF (Peprotech, USA) in 96-well plates. The fractionated CPS-100, unfractionated total CPS (tCPS), LPS (liposaccharide derived from *E-coli* 0111:B4, Invivogen, USA) and medium were added as needed, and cultured at 37°C and 5% CO₂ for 48 hours. According to the manufacturer's instructions, the supernatant was collected after centrifugation and frozen for cytokine estimation via enzyme-linked immunosorbent assay (ELISA, e-Bioscience, Ready set go ELISA kits).

### 2-2. Immune cell co-culture system.

Isolation of spleen cells was performed as described above. CD11c+APCs (Miltenyi Biotec) and naive CD4+ T-cells (Stem Cell Technologies) were isolated in accordance with the manufacturer's protocol. APCs were incubated with probiotic strains at 37°C, 5% CO₂ for 18 to 20 hours. Then, the probiotic strain was washed and were co-cultured with the primed APCs and CD4 + T-cells under specific conditions.

### 3. Safety evaluation.

### 3-1. Hemolysis Test

*L. plantarum* IMB19 was grown under optimal growth conditions, streaked on 5% sheep blood agar (Hanil, Komed) and cultured for 48 hours. Alpha (α) 2 hemolysis was considered as partial degradation of hemoglobin in red blood cells (actual hemolysis does not occur), beta (β) hemolysis observed as a transparent area on agar plates was considered as complete degradation of hemoglobin in red blood cells, and gamma (γ) hemolysis was considered as lack of hemolysis. Bacillus cereus ATCC 27348 was used as a positive control.

### 3-2. Gelatin degradation test

The basic protocol was performed in accordance with the ASM Science Recommendation (Dela Cru et al., 2012). *L. plantarum* IMB19 grown under optimal growth conditions was inoculated onto a gelatin medium with an inoculation loop and incubated at 30°C for up to 5 days, and gelatin liquefaction and bacterial growth were observed daily. Gelatin generally liquefies at 28°C or higher. The tube was stored in the refrigerator for 30 minutes to determine whether or not liquefaction was due to gelatinase activity. Then, the tube was tilted to determine whether or not the gelatin was degraded. When the gelatin is degraded, it appears as a liquefied medium even after cold exposure. Bacillus cereus ATCC 11778 was used as a positive control.

### 3-3. Biogenic amine analysis

*L. plantarum* IMB19 was grown optimally and streaked on a special medium containing the precursor of histamine, cadaverine, tyramine and putrescine in accordance with Bover-Cid and Holzapfel (Bover-Cid et al, 1999) and cultured at 37°C, 30°C and 23°C for 4 days. Then, positive and negative controls were determined by observing the change in medium color. *E. coli* ATCC 25922 was used as a positive control. Culture was performed on agar using a basic culture medium of ornithine, lysine, tyrosine, or histidine, along with a bromocresol purple indicator.

### 3-4. Antibiotic resistance test

The basic protocol was performed based on ISO recommendations (ISO-10932, 2010). The broth dilution method was used to evaluate the minimum inhibitory concentration (MIC) of the strains against the antibiotic.

In culture microdilution, test organisms were grown neat in broth medium and all organisms were washed with 1X PBS. The bacterial solution washed with PBS was adjusted to 600 nm optical density (OD) units of 0.01 to 0.02. A 96 well-plate containing 200 µL of LSM broth medium was inoculated with 10 µL (1-2×10⁵ CFU) of the strain along with antibiotics.

The strain was considered susceptible when it was inhibited at a specific antibiotic concentration that was equal to or lower than the cut-off value set according to the parameters set by the European Food Safety Authority (EFSA, 2018), and the strain was considered tolerable when it was not inhibited at a specific antibiotic concentration that was higher than the cut-off value set according to the parameters.

### 4. Isolation of crude capsular polysaccharide (CPS)

Isolation of crude capsular polysaccharide (CPS) was performed in accordance with a modification mode of a previously described method (Verma et al., 2018). Bacterial cultures were centrifuged and sonicated in a Branson digital sonicator at 10% amplitude and 10 second pulse for 15 minutes. The supernatant was treated with trichloroacetic acid (0.5% w/v) overnight at 4°C. The sample was centrifuged at 6,000 rpm for 20 minutes and 100% ethanol was added to the supernatant at a ratio of 3:1 to precipitate the crude polysaccharide at -20°C. The precipitate was resuspended in Tris buffer (100 mM, Sigma Aldrich, USA) containing magnesium chloride (20 mM, Sigma Aldrich) and calcium chloride (20 mM, Sigma Aldrich) prepared in endotoxin-free distilled water and treated with DNAse (0.1 mg /mL, Roche, Germany) and RNAse (0.4mg/mL, Sigma Aldrich, USA) at 37°C for 4 to 6 hours. To degrade protein contaminants, Pronase (0.3 mg/mL, Sigma-Aldrich, USA) was added to the result, followed by incubation overnight at 4°C. The sample was treated with trichloroacetic acid (1-2% w/v) at 37°C for 30 minutes to remove total protein including added enzyme. The total polysaccharide of the deproteinized sample was re-precipitated by ethanol precipitation. The pellet was resuspended in endotoxin-free water and dialyzed at 4°C for 48 hours with water changes twice a day (MW cut-off 12,000 Da). The total CPS fraction was obtained by lyophilization in a final yield of 20 mg per liter of culture.

### 5. GC-MS analysis conditions

All chemical derivatives were prepared by gas-liquid chromatography (GLC-MS) equipped with a mass selective detector 5973N and a Zebron ZB-5 capillary column (Phenomenex, 30 m × 0.25 mm, *i.d.*, film thickness 0,25 µm, flow rate 1 mL/min, He as carrier gas) using an Agilent 7820A (Santa Clara, CA, USA). Electron impact mass spectra were recorded with an ionization energy of 70 eV and an ionization current of 0.2 mA. The temperature program used was as follows: 150°C for 5 min, 150°C to 300°C at 10 °C/min, 300°C for 12 min.

### 6. NMR Acquisition Parameters

For structural analysis of the isolated polysaccharides, NMR spectra were recorded in D₂O using a Bruker 600 MHz spectrometer equipped with a reverse cryo-probe tilted along the Z-axis. Spectra were measured at 298K or 310K, calibrated with acetone (¹H 2.225 ppm; ¹³C 31.45 ppm) as an internal standard, acquired with Topspin 2.0 software (Bruker), and processed and studied with Topspin 3.6. ¹H-¹H DQ-COSY (double quantum COZY spectrum, hereinafter COSY), TOCSY and NOESY spectra were obtained from a data set (t1 × t2) of 2048 × 512 points and TOCSY and NOESY spectra were collected by scanning 24 times with blending times of 100 ms and 200 ms. Heterogeneous ¹H-¹³C HSQC, HMBC, and HSQC-TOCSY spectra were performed in a ¹H-detection mode using a data set of 2048 × 512 points. HSQC and HSQC-TOSCY were performed with multiple edits in the selection step to distinguish "₂" the density from others. The HMBC was optimized for long-range coupling constants using a lowpass J filter to suppress one-coupled correlations and a 60 ms delay was used for the evolution of long-range correlations. For HSQC-TOCSY, the mixing time was set to 100 ms. In all two-dimensional tests, the data matrix was expanded to 4092 × 2048 points and converted using the qsine or sine window function.

### 7. Animal and Mouse Tumor Models

C57BL/6 & Balb/c mice were obtained and maintained in the Pohang University of Science and Technology animal facility. Pmel-1 TCR transgenic, TLR2 -/-, TLR4 -/-, TLR6 -/-, MyD88 -/- and IL-6 -/- mice were obtained from Jackson Lab and maintained in the POSTECH animal facility. The C57BL/6-derived melanoma cell line B16.F10 and the Balb/c-derived breast cancer cell line EMT-6 were procured from ATCC and maintained by the provided protocol. Syngeneic tumor models were constructed by subcutaneous injection of 200,000 B16.F10 tumor cells or 500,000 EMT-6 cells. Tumor size was measured every other day until endpoint and tumor volume was calculated as a length × width² × 0.5. To analyze the initial infiltration of innate immune cells, tumor cells were subcutaneously injected at 5 mill/mouse. 40 hours later, tumor cells were analyzed. All laboratory animal procedures were performed with the approval of the Animal Care and Use Committee (IACUC) of Pohang University of Science and Technology.

### 8. Cell-based in vitro assay method

Total cells harvested from the spleen and/or lymph node were used for whole spleen cell culture or were applied to magnetic bead isolation (Miltenyi Biotec) to enrich dendritic cells, naive CD8+ T-cells or naive CD4+ T-cells. A total of spleen cells (200,000/well) and bacteria were cultured at a ratio of 1:1 in a 96-well plate and the supernatant was harvested at 48 hours and used for ELISA (eBioscience Ready set Go kits). CD11c+ dendritic cells (200,000/well) were primed with an appropriate ratio of bacteria for 18 to 20 hours and washed, and then T-cells (200,000/well) were added thereto, followed by culture for 72 hours. As indicated, anti-CD3@ 0.01 ug/ml (BioXCell) and GM-CSF@ 2.5 ng/ml (Peprotech) for stimulation of CD8+ T cells, and anti-CD3@0.1 µg/ml, GM-CSF@10ng/ml, IL2@100U/ml and TGFβ for stimulation of CD4+ T cells were added.

In peritoneal macrophages, the cells were harvested 5 days after intraperitoneal injection of 2% Biogel (Bio-Rad) and *in vitro* cultured along with synthetic murine MCSF 10 ng/ml (Peprotech). For polarization of selectively activated macrophages, IL-4 (Peprotech) was added thereto for 24 hours, and treated with CPS, LPS (*E-coli* 0111:B4-derived lipopolysaccharide, Invivogen) or Pam3CSk4 (Sigma).

### 9. Metagenome analysis and whole genome sequencing

Fecal pellets from tumor-bearing SPF mice were consigned for metagenome analysis (Macrogen, S. Korea) . Whole-gene sequencing of bacteria was performed in the Illumina platform (Macrogen, S.Korea); Bacterial culture samples were sent directly for analysis. Bioinformatic analysis was also consigned to Macrogen.

### 10. Classification and gene expression profile analysis of tumor-infiltrating macrophages

Mice were injected with 500 µg of CPS twice at 24-hour intervals, and then 5×10⁶ B16.F10 tumor cells were inoculated subcutaneously. 40 hours after tumor implantation, whole tumors, including infiltrating immune cells, were digested in Liberase into single cell suspensions. Samples of 5 to 10 mice were recruited from the same treatment group, and stained with Fixable Viabilityef506 (eBioscience), CD45-AF488 (Bioloegend, 30-F11), CD3-ef450 (Ebioscience, 145-2C11), CD19-PB (Ebioscience, 1D3), I-A/I-E-PECy7 (Biolegend, M5/114.15.2), CD11c-PE (Ebioscience, N418) and CD11b-PerCpCy5.5 (BD, M1/70). Live CD45⁺ CD3⁻ CD19⁻ MHCII ^{hi}CD11c⁺ CD11b⁺ macrophages were sorted with FBS-supplemented medium, centrifuged and stored in Trizol (Sigma). Samples were analyzed for gene expression profiles in Macrogen, the average fold-change of gene transcript numbers between treatment groups was calculated, and genes with a fold-change of 1.5 or more in the two comparisons were input to DAVID v6.7 (The Database for Annotation, Visualization and Integrated Discovery v6.7) for pathway analysis. Genes determined to have significantly enhanced immune function were marked on a heat map (p<0.05).

### 11. Listeria monocytogenes in vivo cytotoxicity assay

Analysis was performed in accordance with a conventionally known method. Specifically, Listeria monocytogenes expressing the OVA peptide (LM-OVA) were injected into C57/Bl6 mice at 5,000 CFU/mice. The mice were fed *L. plantarum* IMB19 strain every other day. On day 6, splenocytes from naive C57/bl6 mice were pulsed with OVA peptide, and cells with or without peptide pulse were stained with CFSE or CTV and mixed at a ratio of 1:1, and the mixture was administered intravenously to the infected mice. After 2 hours, mice infected with LM-OVA were sacrificed, splenocytes/lymph nodes were harvested therefrom and apoptosis of peptide-pulsed splenocytes was detected using flow cytometry.

### 12. Purification using chromatography

The entire isolated capsular polysaccharide (28 mg of tCPS) was purified using an anion exchange Q-Sepharose high-speed flow method (GE Healthcare; V = 4.4 mL, flow 16 mL/h). The resin was packed, washed with 1 M NaCl, and equilibrated with 10 volumes of NaCl 10 mM. Then, total capsular polysaccharide (tCPS) was dissolved in 10 mM NaCl (5 mL) and adsorbed onto the resin. Elution was performed serially by sequentially adding 16 mL of NaCl (10, 100, 200, 400, 700 and 1,000 mM, respectively). The eluate eluted at each concentration of NaCl was collected, desalted by dialysis (cut-off 1 kDa) and lyophilized. Six fractions eluted at each concentration of NaCl were labeled with CPS-X (X is the concentration of NaCl used for elution, mM).

The molecular weight of CPS-100 was measured using an HPLC system Agilent 1100 using a TSK G-5000 PWXL size exclusion column (30 cm × 7.8 mm) equilibrated with 50 mM NH₄ HCO₃ as an eluent (flow = 0.8 mL/min) and the eluate was monitored with a refractive index detector. The column was calibrated by injecting a dextran standard (50 µL of a 1 mg/mL solution) with a known molecular weight (12, 50, 150 and 670 kDa each). The logarithm of the molecular weight was plotted against the elution volume and the MW of the polysaccharide was calculated using an established linear relationship (LogPM=-0.811mL+11.7; R2=0.98).

### 13. QUANTI-Blue SEAP analysis.

HEK293 cells transfected with human TLR 2 (InvivoGen) having an inducible SEAP reporter gene were cultured in 200 ml DMEM (complete growth medium) supplemented with 10% fetal bovine serum (FBS), 1% penicillin/streptomycin, and 1% L-glutamine. Specifically, cells were seeded in a 96-well plate at 70,000 cells/well and cultured at 37°C in complete growth medium for 24 hours. The medium was removed from the 96-well plate, was replaced with DMEM supplemented with 10% FBS (containing no 1% penicillin/streptomycin, and 1% L-glutamine) and then treated with an appropriate concentration of compound. Then, the cells were incubated overnight at 37°C for 18 to 24 hours. To detect SEAP activity, 20 mL of medium was removed from each well, transferred to a transparent 96-well plate containing 180 ml of QUANTI-Blue reagent (InvivoGen), and incubated in the dark at 37°C for 30 minutes to 2 hours.

### 14. TCR repertoire analysis

Tumor-infiltrating CD8+ T cells were classified into CD45⁺ Dump⁻ CD3⁺ CD8a⁺ (Dump CD19/CD11c/Ly6c/Ly6G/CD11b) using a MoFlow sorter (BD biosciences) on days 18 to 20 of the experiment. The cells were collected in HEPES/PBS buffer and centrifuged and cell pellets were stored at -80°C until DNA isolation (AbCam). DNA from 4 to 5 mice was pooled into a single sample and commissioned for TCR-β repertoire analysis (Adaptive Bio). Data were analyzed on the Immunoseq platform provided by Adaptive Bio.

### 15. Statistical Analysis

Tumor growth curves were plotted by two-way ANOVA using Sidak's multiple comparison post-test for comparison of two groups, Dunnett's multiple comparison post-test for comparison between multiple groups and controls, or Tukey's multiple comparison post-test for individual comparison of two or more groups. For other comparisons, unpaired Student's t-test was used for comparison between two groups, and Bonferroni correction for multiple test and one-way ANOVA were used for comparison between two or more groups. P<0.05 was considered statistically significant (*P<0.05, **P<0.01, ***P<0.001, ****P<0.0001). Statistical analysis was performed using GraphPad PRISM v8.0. Flow cytometry data were analyzed using Flow-jo software v10.1.

### Example 2: Isolation and identification of L. plantarum IMB19

*Lactobacillus plantarum* IMB19 strain KCTC14337BP was mainly isolated from domestic kimchi fermented by raw material-derived microorganisms. To induce colonies of *Lactobacillus sp.*, serially diluted kimchi suspensions were streaked onto MRS broth (De Man, Rogosa and Sharpe broth, Becton-Dickinson, USA) plates. Cultured single colonies were isolated and further cultured in MRS broth. Since the morphology of colonies was not sufficient to distinguish strains from each other, 14 isolated bacteria were subjected to PCR and 16s rRNA sequencing and sequence similarity was identified using NCBI's BLAST. All of the isolated bacteria were found to belong to lactic acid bacteria (LAB). Among the isolated bacteria, strains with more than 99% similarity with *L*. *plantarum* were identified and the strain was found to have 99% similarity with many isolated bacteria agar *Weissella koreensis.* These results are consistent with the previous report that *L*. *plantarum* and *Weissella koreensis* are the dominant species of kimchi.

The analyzed 16S rRNA sequence information of *L. plantarum* IMB19 is as follows.
- ***L*. *plantarum* IMB19 16S rRNA (785 Forward) (SEQ ID NO: 3)** 
AGCGCTGGGATGATGCTAGTGTTGGAGGGTTTCCGCCCTTCAGTGCTGCAGCTAACGCAT
TAAGCATTCCGCCTGGGGAGTACGGCCGCAAGGCTGAAACTCAAAGGAATTGACGGGGGCCCGCA
CAAGCGGTGGAGCATGTGGTTTAATTCGAAGCTACGCGAAGAACCTTACCAGGTCTTGACATACT
ATGCAAATCTAAGAGATTAGACGTTCCCTTCGGGGACATGGATACAGGTGGTGCATGGTTGTCGT
CAGCTCGTGTCGTGAGATGTTGGGTTAAGTCCCGCAACGAGCGCAACCCTTATTATCAGTTGCCA
GCATTAAGTTGGGCACTCTGGTGAGACTGCCGGTGACAAACCGGAGGAAGGTGGGGATGACGTCA
AATCATCATGCCCCTTATGACCTGGGCTACACACGTGCTACAATGGATGGTACAACGAGTTGCGA
ACTCGCGAGAGTAAGCTAATCTCTTAAAGCCATTCTCAGTTCGGATTGTAGGCTGCAACTCGCCT
ACATGAAGTCGGAATCGCTAGTAATCGCGGATCAGCATGCCGCGGTGAATACGTTCCCGGGCCTT
GTACACACCGCCCGTCACACCATGAGAGTTTGTAACACCCAAAGTCGGTGGGGTAACCTTTTAGG
AACCAGCCGCCTAAGGTGGGACAGATGATTAGGGTGAAGTCGTAACAGGGTAAAACCGTAAAGAT
GTTCAACCCGCCACATCTGTCGCGTCTCCGTCGTAGATATAAGAAAGCCAAAGGGCCTTTCTTCC
 
ATGGCTGGGTGTTCATGCAATAACATCGACCGGTTATCCACGACACAAGAAAGGATTACGTTGGT

CCTGGTTGTGCGCTCAGGTTTTATAGTGACAGCGGGCCTATTTGTATGGTGTAAACCGGAGTGCT

AACAATCTTCTACAAGAAACAGCCTGTACATAAATTTACGGCATATATATACCGGAACGTGGCTT

GGCCACGTATGTTATTAACGCGGGCTGGCAGGAACTTACTAGGCCGTGCCATTCCGGTGTCAAAT

CCGACCGAATCCGGGGACTCGTCTCGCGGAAATGTGTTTCTTTTTAGAGACATGGATTCTTACAA

ACCGAGACCCTGTCATGCCCGGGATGAGGGTCTGCCACTAACAACTTTCCGAACATGATGGGAAG

AACCCCCTAACGGGCGCCCACCTGGAGGAATTTGGGCCGGGGCACCACCGCCCGAGGTGGGGCGG

AAAACCCCCTCCAGGGGTCCCATCCTCAATTTTTCCGGGGGGGACCCCCCTCCCCCCCAAAATGA

GGGAAAACCCCCGGGGGGGCACCCCCAAAAGAAGGAGAGCCCCCCACCCTCACTCTTCCCGCCCG

GCGTGCGGGGGCGGGTTTTTTTTTCTGTCAAAATAAATTTTGTGTTGTTTGTGTGTTCCTCCCCC

CCCCGCCGCGGGGGCGGGGTTGTACTTTTTTCCCTCTCCATCCCCCCCCCACCACAAAAGAAAAG

GAGGGGACGACACCCACAGTGGGTGTGTTTTT
- ***L*. *plantarum* IMB19 16S rRNA (907 Reverse) (SEQ ID NO: 4)**

TTGACGGGGGGGTCTCCAGGCGGAATGCTTAATGCGTTAGCTGCAGCACTGAAGGGCGGA
AACCCCCCAACACTTAGCATTCATCGTTTACGGTATGGACTACCAGGGTATCTAATCCTGTTTGC
TACCCATACTTTCGAGCCTCAGCGTCAGTTACAGACCAGACAGCCGCCTTCGCCACTGGTGTTCT
TCCATATATCTACGCATTTCACCGCTACACATGGAGTTCCACTGTCCTCTTCTGCACTCAAGTTT
CCCAGTTTCCGATGCACTTCTTCGGTTGAGCCGAAGGCTTTCACATCAGACTTAAAAAACCGCCT
GCGCTCGCTTTACGCCCAATAAATCCGGACAACGCTTGCCACCTACGTATTACCGCGGCTGCTGG
CACGTAGTTAGCCGTGGCTTTCTGGTTAAATACCGTCAATACCTGAACAGTTACTCTCAGATATG
TTCTTCTTTAACAACAGAGTTTTACGAGCCGAAACCCTTCTTCACTCACGCGGCGTTGCTCCATC
AGACTTTCGTCCATTGTGGAAGATTCCCTACTGCTGCCTCCCGTAGGAGTTTGGGCCGTGTCTCA
GTCCCAATGTGGCCGATTACCCTCTCAGGTCGGCTACGTATCATTGCCATGGTGAGCCGTTACCC
CACCATCTAGCTAATACGCCGCGGGACCATCCAAAAGTGATAGCCGAAGCCATCTTTCAAACTCG

GACCATGCGGTCCAAGTTGTTATGCGGTATTAGCATCTGTTTCCAGGTGTTATCCCCCGCTTCTG
GGCAGGTTTCCCACGTGTTACTCACCAGTTCGCCACTCACTCAAATGTAAATCATGATGCAAGCA
CCAATCAATACCAGAGTTCGTTCGACTTGCATGTATTAGGCACGCCGCCAGCGTTCGTCCTGACA
GAGAGAAAAAAAAAAAAAAAAAAGGGCCGGGGGGATCGGGGGGGGGGGGGGGGGGGGTGAGGGGT
TGAGGGGGGGGGGGGGGGGGGGGGGGGGGGGGGGGGGGGGGGGGGGGGGGGGGGGGGGGGGG
GGGGGGGGGGGGGGGGGGGGGGGGGGGGGGGGGGGGGGGGGGGGGGGGGGGGGGGGGTGTGTG
GGGGGGGGGGGGTTGTTGTTTTTGTTTGGGGGGGGGGTTGTTTTTTGTGTGTGTTTTGTTGTTTG
TTTGGGGGTGTGTTTTGTTGTGGGGTGGGGTGTTGGGGGGGTTGGGGGGGGGGTGTTGTTTGGGG
GGGGTGGGGGGGGGGGTTTTTTTGTTGTTGTGTGGTTGTGTGTTGTGTGGTGGGTGGGGGGGGTG
GTGTGTGTGTGGGGGTGGGGGGTGTTTGGTGGGGGGGGGGTTGTTGTGGGGGGGTGGTGTTTGTT
TTTTGTTTTTTTTTGTGTGTGGGGGGGGGGGGTGGGGGGTGGTTTGTGGGGTGTTGTTTGTGTGT
GGTTGGTGGTGGTGTGTGGGGGGGTTGGGGGGGGGGGGGTTGTCTTTTTTGTTGGTGTTGGGTGT
TTGTTGGTGTTGGTGTGTGGTGGGGTGGTGTGGTGGGTGGGTGCTTGTTGTGTGTGTGGTGTGT

### Example 3: Selection of L. plantarum IMB19

In order to determine the immunostimulatory effect of the isolated bacteria on immune cells, the effect of the bacterial culture on murine splenocytes was tested to determine bacteria having a stimulatory effect on immune cells. Whole splenocytes were cultured with the isolated bacteria for 48 hours and then the level of cytokines in the culture supernatant was measured. The effects of the isolated bacteria on immune cells were evaluated using IFN-γ, which is a well-known inflammatory marker, and IL-10, which is an anti-inflammatory cytokine. Among all the isolated bacteria, a novel *Lactobacillus plantarum* strain that induces a negligible level of IL-10 and very high levels of IFN-γ was selected and was designated "*L. plantarum* IMB19" (FIG. 1, colony 1).

### Example 4: Microbiological and biochemical characterization of L. plantarum IMB19

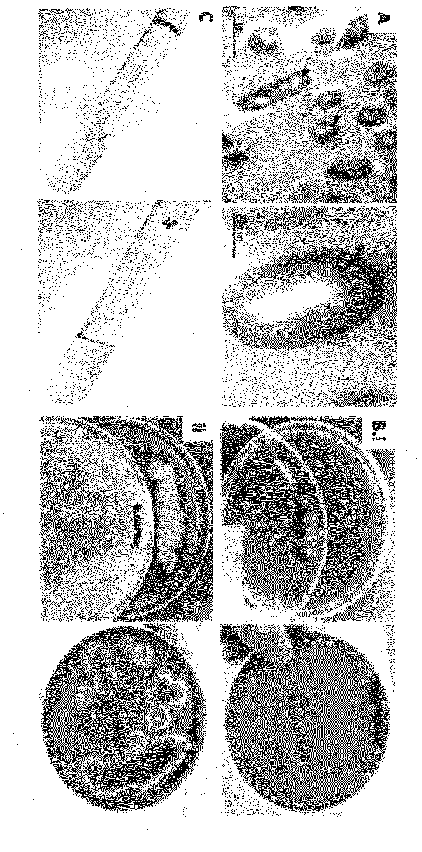

Colonies of *L. plantarum* are small, smooth, round, and translucent. Non-flagellated, rod-shaped microbial colonies of *L*. *plantarum* were identified by cryosection TEM (FIG. 2A). A thick capsular layer of each bacteria was usually clearly identified. When cultured on sheep blood agar, there was no transparent or green area, which indicates that the strain was non-hemolytic or γ-hemolytic (γ), like other *Lactobacilli* (FIG. 2B). *Bacillus cereus* ATCC 11778, which was used as a positive control, exhibited negative gelatinase activity up to day 5 (FIG. 2C). In addition, the production of four biogenic amines (histamine, cadaverine, tyramine and putrescine) of the strain was tested. *L. plantarum* IMB19 was found not to produce biogenic amines, unlike *E-coli* ATCC 25922 (Table 1).

**[Table 1]**

| Biogenic amine production activity of *L. plantarum* IMB19 and *E. coli* ATCC 25922 | | | | |
|---|---|---|---|---|
| **Strain** | **Histamine** | **Cadaverine** | **Tyramine** | **Putrescine** |
| *L. plantarum* IMB19 | Negative | Negative | Negative | Negative |
| *E. coli* ATCC 25922 (positive control) | Positive | Positive | Positive | Positive |

As shown in Table 2 below, the antibiotic susceptibility test results showed that, like most *Lactobacillus* species, the strains exhibit sensitivity to most clinically relevant antibiotics excluding kanamycin (Appl Environ Microbiol 85, (2019)).

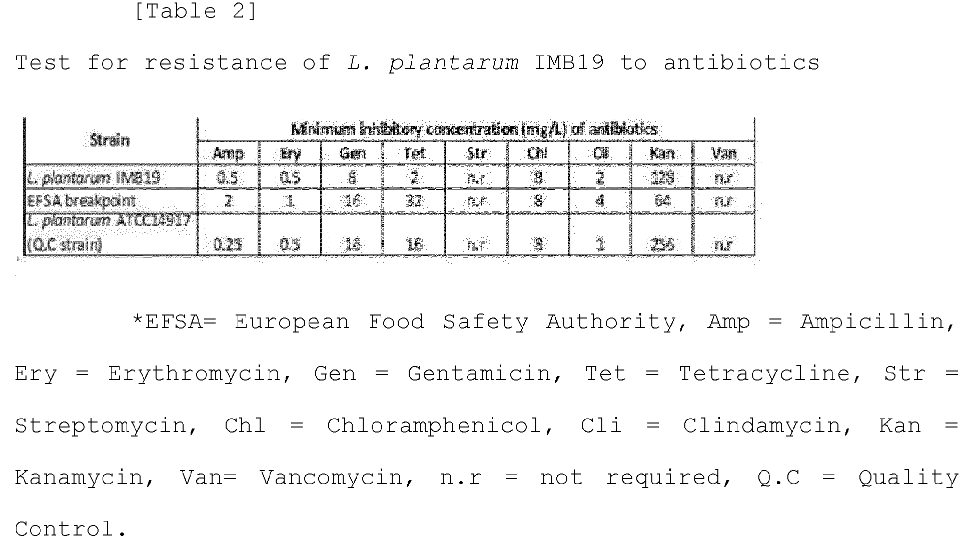

### Example 5: Genetic characterization of L. plantarum IMB19

Sequence similarity comparison based on 16S rRNA sequence analysis confirmed the identity between *L. plantarum* species. In addition, the *L. plantarum* IMB19 was distinguished from other genetically related species such as *L. pentosus* and *L. paraplantarum* through recA gene analysis by PCR (Appl Environ Microbiol 67, 3450-3454 (2001)).

Analysis based on DNA isolation, Pac-Bio & Illumina Hi-seq sequencing and bioinformatics was performed (Macrogen, Korea).

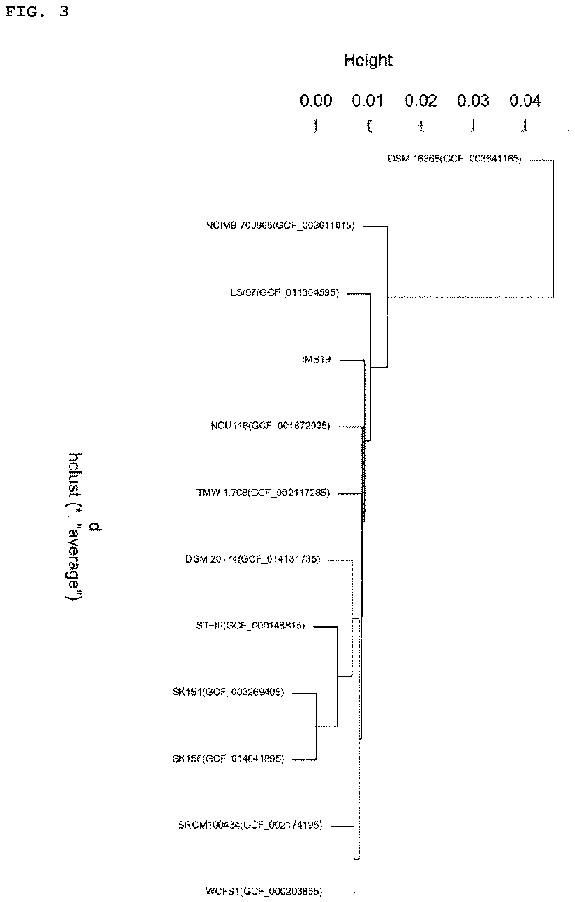

*L. plantarum* IMB19 was distinguished from other genetically closely related species by amplifying recA genes using recA genederived primers and comparing bands. Since several *L. plantarum* strains are known to have similarities in the entire genome, phylogenetic analysis was performed based on the average nucleotide index (OrthoANI), and *L. plantarum* IMB19 was identified as a unique strain (FIG. 3). The presence of putative pathogenic genes was identified using Virulence Finder 2.0 based on the pathogenic gene sequence homology between four known strains. No significant hits to pathogenic genes were seen using the 90% nucleotide cut-off. As can be seen from ResFinder (J Clin Microbiol 52, 1501-1510 (2014)), there is no antibiotic-resistant gene in the entire genome of *L*. *plantarum* IMB19.

As disclosed in the examples above, *L. plantarum* IMB19 is a novel strain having different characteristics from previously reported *L. plantarum* strains and was deposited under accession number KCTC 14337BP on the Korea Research Institute of Bioscience and Biotechnology on October 21, 2020.

### Example 6: Immunostimulatory effects of L. plantarum IMB19 on murine T cells.

The beneficial effects of several strains belonging to *L*. *plantarum* on host immunity and health have been previously reported (Biomed Res Int 2018, 9361614 (2018)), and the direct effect of the *L. plantarum* IMB19 strain of the present invention on murine immune cells was determined. The present inventors expected that uptake of bacterial antigens by antigen-presenting cells (APCs) might alter the activation state, thereby priming other immune cells and consequently modulating the immune system. Therefore, a co-culture system containing both APCs and CD4+T cells representing the innate and adaptive immune system was designed. CD11c+APCs were exposed to bacteria for 20 hours (APC : bacteria = 1 : 100). These APCs did not distort the immunophenotype and were co-cultured with naive CD4+T cells under suboptimal external stimulation. To identify the differentiation of T-cells into Th1, Th2, Th17 or regulatory T cells (Treg), other transcription factors, Tbet, GATA3, RORγ and Foxp3 were analyzed. Without any external influence, *L*. *plantarum* IMB19 markedly induced the production of CD4+ RORγTh17 cells as compared to the other three strains (FIG. 4A). Whether or not other Th cell subtypes were significantly produced was not clearly detected and they were similar to other *L. plantarum* strains (FIG. 4B).

To verify the above results, the production of Th17 cells by *L. plantarum* IMB19 was tested under minimal Th17 cell production conditions. *L. plantarum* IMB19 significantly increased the production of CD4+ RORγTh17 that produces great amounts of IL-17 (FIG. 5A).

On the other hand, *L. plantarum* IMB19 did not induce significant amounts of Foxp3 under neutral conditions (FIG. 5B). Therefore, whether or not *L. plantarum* IMB19 could produce Tregs from naive CD4+ T- cells in sufficient concentrations of TGF-β was determined. The result showed that the generation of Treg was significantly suppressed under the increased concentration of TGF-β.

### Example 7: Confirmation of L. plantarum IMB19 promoting anti-tumor immune response in vivo

*L. plantarum* IMB19 strain and various *Lactobacillus* strains (14 strains) as controls were cultured along with spleen cells to compare changes in cytokine production by immune cells. The result showed that, compared to other *Lactobacillus* strains, *L. plantarum* IMB19 exhibited significantly higher IFN-γ levels and significantly lower IL-10 levels (FIG. 6A). For reference, another strain, *Lactobacillus murinus*, exhibited the highest ability of inducing IL-10 and low IFN-γ production (FIG. 6A).

Since CD8+ T-cells are the main anti-tumor effector cells, the CD8+ T cell stimulating ability of the two strains was determined. In the co-culture of dendritic cells (DC) and CD8+ T cells primed with bacteria, an IFN-γ level was increased when treated with *L. plantarum* IMB19, especially, an IFN-γ level was significantly increased compared to when treated with *Lactobacillus murinus* (FIG. 1B). Activation of CD8+T-cells upon co-culture with splenic and mesenteric lymph node antigen-presenting cells varied in a bacterial-concentration dependent manner (FIGS. 7A and 7B).

*L. plantarum* IMB19 did not directly stimulate CD8+ T-cells in the absence of dendritic cells. Activation of TCR-delivering CD8+T- cells to the mouse melanoma-specific antigen Pmel-1 was similarly activated in the presence of the antigen gp-100 (FIG. 7C). In addition to DC, macrophages are APCs known to play an important role in tumor growth and suppression. Therefore, the effects of *L. plantarum* IMB19 on CD8+ T-cell stimulation through CD11b+ F4/80+ peritoneal macrophages was determined. Similar to dendritic cells, *L. plantarum* IMB19 greatly increased the proportion of IFN-γ cells during macrophage-CD8+T-cell co-culture (FIG. 6C).

CD4+Foxp3+ regulatory T cells (Tregs) accumulate in tumors and suppress the effector functions of CD8+ T cells and other immune cells to promote tumor progression. In addition, several bacteria that promote Treg production have been reported (Nature 453, 620-625 (2008); Sci Immunol 3, (2018)). Therefore, the present inventors determined whether or not *L. plantarum* IMB19 affects Treg induction in the co-culture of CD11c+ DC and CD4+T- cells. Under highly Treg distortion culture conditions, *L. plantarum* IMB19 exhibited significant suppression of Treg production in the presence of TGF-β (FIG. 6D). This effect was maintained even at lower levels of TGF-β and no Treg generation was observed in any of the conditions tested (FIG. 8A). On the other hand, interleukin-17A (IL-17A) increased (FIG. 8B), which indicates the production of T-helper-17 cells (Th17). Interleukin-6 (IL-6) is essential for Th17 production and suppresses Treg production in the presence of TGF-β (Eur J Immunol 40, 1830-1835 (2010)). Accordingly, co-culture of IL-6-deficient DCs with CD4+ T cells generated Tregs. Thus, IL-6 production by *L. plantarum* IMB19 primed DCs suppresses Treg production under Treg-distorting culture conditions.

To test whether or not CD8+ T cells activated by *L*. *plantarum* IMB19 are functionally cytotoxic, cytotoxicity was tested using an acute *Listeria monocytogenes* infection model expressing OVA antigen (LM-OVA) (FIG. 6E). LM-OVA-infected mice fed *L*. *plantarum* IMB19 exhibited a significant increase in lysis of target cells loaded with OVA-pulsed CFSE *in vivo* (FIG. 6F). In addition, the effect of *L. plantarum* IMB19 on subcutaneously transplanted B16.F10 melanoma in both SPF (specific pathogen free) and GF (germ free) mice was evaluated (FIG. 6G). *L. plantarum* IMB19 significantly inhibited melanoma growth in both SPF and GF mice, compared to *L. murinus* (FIGS. 6H and 6I). To evaluate whether or not *L. plantarum* IMB19 induces any dysbiosis leading to antitumor effects, 16s ribosomal RNA sequencing was performed on fecal samples from tumor-bearing animals fed *L. plantarum* IMB19. However, no significant changes in microbial diversity were found in the feces of *L. plantarum* IMB19-fed mice compared to the PBS control group (FIGS. 9A to 9C). This indicates that *L. plantarum* IMB19-mediated regulation of antitumor immunity corresponds to *L*. *plantarum* IMB19-specific effects and is not a result of colony collapse. In conclusion, the data in this example indicate that *L*. *plantarum* IMB19 is a positive regulator of cytotoxic T cellmediated antitumor immune responses *in vivo.*

### Example 8: Chemical characterization of crude polysaccharide fraction derived from L. plantarum IMB19

The results of analysis by transmission electron microscopy (TEM) showed that the cells of *L. plantarum* IMB19 were surrounded by a layer of capsular material containing a carbohydrate composition containing rhamnose, galactose, glucose and glucosamine, and small amounts of glycerol and ribitol. (FIGS. 10 and 11A). The two polyols are usually related to the presence of teichoic acids, which are interconnected through phosphodiester bonds (Tomita, Tanaka, & Okada, 2017). In general, it is very difficult to detect these polyols because methanolysis cannot completely cleave the phosphodiester bonds. Therefore, the GC-MS analysis was repeated by dephosphorylating the sample with aqueous HF prior to methanolysis and acetylation, and the presence of teichoic acids is detected based on the increasing amounts of both polyols. In monosaccharides, rhamnose has an L absolute configuration, and glucose and galactose have a D configuration (FIG. 12). On the other hand, glucosamine was assumed to have a D configuration based on the exclusive presence of stereoisomers.

### Example 9: Purification of crude polysaccharides

The results of chemical analysis of the carbohydrate component suggested that CPS may be a mixture of polymers. Accordingly, six fractions obtained by purifying CPS using ion exchange chromatography were expressed as CPS-X depending on the concentration (X) of the eluate used. Yields obtained through purification were as follows: CPS-10 11%, CPS-100 13%, CPS-200 9.0%, CPS-400 51%, CPS-700 7.0%, and CPS-1000 7.9%. Each fraction was compared with the spectral profile of the original mixture (CPS) using ¹H NMR analysis (FIG. 13).

Specifically, each fraction is as follows (FIG. 13).

CPS-10 (FIG. 13B): CPS-10 had a rather heterogeneous spectrum. The anomeric region of CPS-10 contains two major signals with strong signals at 4.2 and 1.3 ppm related to carbohydrates and other substances.

CPS-100 (FIG. 13C): The anomeric region (5.6-4.5 ppm) of CPS-100 contains 9 main signals with double doublets (FIG. 14) at 5.5 ppm that have α-configured (³ *J*_{H1,H2}=3.4 Hz) and phosphorylated (³ *J*_{H1}, P=7.0 Hz) residues. In addition, strong signals including several methyl groups of acetyl groups (methyl groups at 2.06 ppm) and deoxy residues (1.3 ppm) consistent with the presence of N-acetylglucosamine and rhamnose units, respectively, were identified.

CPS-200 (FIG. 13D): CPS-200 was a mixture of CPS-100 and CPS-400, and exhibits four rather broad signals in the anomeric region, and intense methyl signals at 1.6 ppm (not matching a methyl of rhamnose) and in a congested kAminol region (4.4-3.2 ppm).

Integration showed that the ratio between anomeric protons and carbinol protons was 1.0:12, and the generally expected ratio was 1:6 or less, which indicates that CPS-400 does not have the typical structure of polysaccharides because the increase in the ratio is due to the presence of ribitol and glycerol units, as can be seen from chemical analysis (FIG. 15). In addition, glucose was the most abundant monosaccharide, followed by two monosaccharides, glucosamine (GlcN) and muramic acid (MurA) in small amounts as characteristics of peptidoglycan.

This observation indicates that CPS-400 is a teichoic acid (TA) .

CPS-700 and CPS-1000 (FIGS. 13F and 13G): The anomeric region contained only a portion of the signal found in CPS-400, and the ratio between anomeric protons and carbinol protons in both fractions was atypical. Signals from non-carbohydrate substances were also further confirmed.

The amounts of CPS-10, CPS-700 and CPS-1000 in CPS were not great and were only small.

### Example 10: NMR analysis of CPS-100

The structure of the capsular polysaccharide was determined by analyzing the complete set of ¹H-¹H homonuclear (COSY, TOCSY, NOESY) and ¹H-¹³C heteronuclear (HSQC, HMBC, HSQC-TOCSY) 2D NMR spectra recorded at 310 K (Table 3).

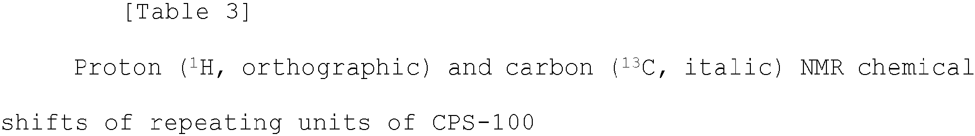

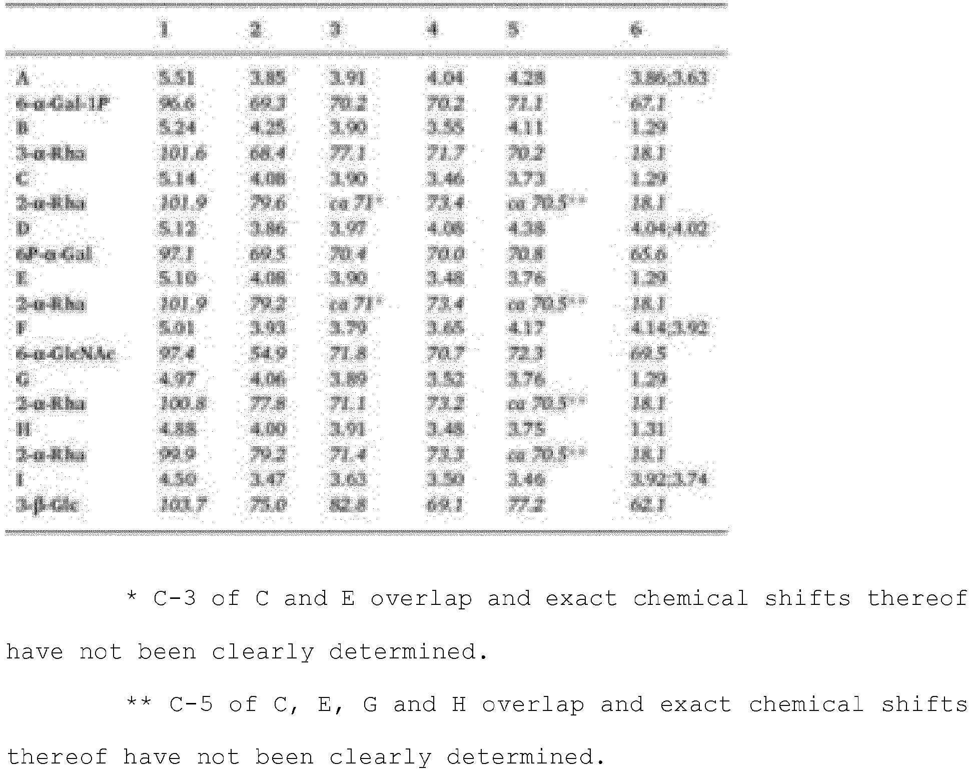

An increase in the temperature from 297 K (FIG. 13C) to 310 K (FIG. 14) reduced the overlap between the three anomeric signals at about 5.15 ppm and the simplified NMR properties of related residues.

The HSQC spectrum (FIG. 14) showed 9 major anomeric densities in ¹H 5.6-4.5 indicated by capital letters A-I, FIG. 14A, and Table 3, and the corresponding protons all had similar proportions. The NMR analysis started at H-1 of A (5.51 ppm) showing three correlations in the TOCSY spectrum, in common with 3.85 ppm in the COZY spectrum (FIG. 16). Thus, this density corresponded to H-2, and in a similar approach, specific densities also corresponded to H-3 (3.91 ppm) and H-4 (4.04 ppm). Since there was no further correlation with H-1, A was identified as a galactose unit. H-5 was identified in the NOESY spectrum due to the strong H-4/H-5 correlation (FIG. 17), and two H-6 were identified through the corresponding COZY correlation (FIGS. 16 and 17).

In HSQC and HSQC-TOCSY spectra (FIG. 18A), all carbon chemical shifts of A (Table 3), which is an α-galactose unit linked to O-6, were defined based on the high chemical shift of C-6 (67.1 ppm). Since the correlation of the TOCSY spectrum occurring at H-1 (5.12 ppm) of D has the same pattern as A, D is galactose, α is constructed based on the ³ *J* _{H1}, _{H2} (3.9 Hz) value, and the chemical shift of H-6s/C-6 (4.04-4.02/65.6 ppm) indicates that phosphorylation occurs consistent with data from previous literature at this position (Sechenkova et al., 2004). Thus, D was 6P-α-Gal and was not branched further based on the high field values of all other carbochemical shifts.

For B, H-1 (5.24 ppm) showed the strongest two TOCSY correlations with H-2 (4.25 ppm), which corresponds to the COZY density (FIG. 16). TOCSY reading on this second proton (FIG. 16) identified all other protons of the unit containing methyl at 1.28 ppm, which is a full diagnosis of the pattern of rhamnose residues. Thus, B has an α-configured rhamnose at the anomeric center based on the similarity of C-5 value (ca. 70.5 ppm) to the reference glycoside (69.4 or 73.6 ppm for α- or β-methylglycosides, respectively; Bock & Pedersen, 1983) and is 3-substituted as defined from the glycosylation shift undergoing at that carbon. The TOCSY pattern of residues C, E, G, and H (H-1 at 5.14, 5.10, 4.97, and 4.88 ppm, respectively) was similar to that of B, indicating that it was an α-rhamnose unit. The lower field value of C-2 (77.8-79.6 ppm) compared to the reference value (71.0 ppm, Bock & Pedersen, 1983) means substitution at O-2.

In F, H-1 (5.01 ppm) had 4 TOCSY correlations (FIG. 16), which were attributed to H-2 to H-5 in the COZY spectrum, and is a pattern of the gluco-configurated residue. HSQC-TOCSY analysis of H-5 confirmed a correlation with density at 69.5 ppm, which was attributed to C-6 and in turn related to H-6s (4.14 and 3.96 ppm). Thus, F is N-acetylglucosamine based on the values of C-2 (54.9 ppm) and H-2 (3.93 ppm) and substituted at C-6 (69.4 ppm). The α configuration was inferred from the shape of the anomeric signal (broad singlet) and the C-3 value (71.8 ppm), which is very similar to the reference α-glycoside (72.0 ppm, Bock & Pedersen, 1983). Finally, H-1 of I (4.50 ppm) had a TOCSY pattern representing all protons in the unit. Therefore, based on the ¹³C chemical shift, I is substituted at C-3 (82.8 ppm), and β-configured glucose based on the ³*J* _{H1,H2} (7.9 Hz) .

Sequences between the residues were deduced by analyzing the HMBC (FIG. 5b) and NOESY (FIG. 4) spectra. First, the correlation and corresponding density of HMBCs connected to H-1 of B and C-3 of I were indicated as B₁I₃ (FIG. 18B). Other correlations such as C₁E₂, D₁B₃, F₁G₂, H₁A₆ and I₁F₆ were found in the same format. In addition, H-1 of E and H-1 of G had a long-range correlation with carbon at about 79 ppm, similar to C-2 of C and H. The exact assignment of these densities to E₁H₂ and G₁C₂ was deduced by analyzing the NOESY spectra (FIG. 17) involving H-1 of E and H-2 of H and H-1 of G and H-2 of C. This property was identified by observing the decorrelation detailed in the expansion of H-2 protons of different rhamnose units (FIG. 19).

Finally, the information that A and D are phosphorylated via phosphodiester bonds at O-1 and O-6, respectively, explains why there is no HMBC linkage for H-1 of A and C-6 of D, or H-6 of D and C-6 of A, or why these protons do not have NOE correlations between residues. Because the HMBC spectrum actually has a large number of bonds (five linkages) between H-1 (or C-1) of A and C-6 (or H-6) of D that exceed the limit of this sequence (3 linkages), no correlation between the two units could be detected. Similarly, the density of the phosphate moiety between A and D keeps the protons of these units very far apart to provide a detectable NOE effect. Therefore, the repeating unit structure of CPS-100 is nonsaccharide as shown in FIG. 20.

In addition, a minor NMR signal was investigated to understand characteristics thereof. In the HSQC spectrum (FIG. 14), the carbon chemical shifts of the densities indicated by ¹H/¹³C 5.26/93.5 and 4.57/97.8 ppm for Gala and Galβ, respectively, represent α or β residues in free reduced form. The TOCSY spectrum in H-1 (FIG. 16) showed a typical pattern of galacto constituent sugars (i.e., for Gala, the third density almost overlapped the COZY density). This finding involved sonication and trichloroacetic acid treatment for the separation of CPS. This is based on the fact that during both treatment processes, particularly, in the anomeric phosphate of galactose A, cleavage of some of these bonds may be induced due to the extreme flexibility of the phosphodiester bonds. Therefore, the minor signal next to the strong signal (indicated by "*" in FIG. 14) belonged to the first repeating residue and was expected to be linked to galactose in a free reduced form. However, the low intensity thereof and high concentration of carbinolic regions prevented accurate characterization. Finally, when the anomeric density is integrated in the HSQC spectrum (FIG. 14A), the sample has an average degree of polymerization of 4 and an average MW of about 6 kDa (MW of a repeating unit is 1,500 Da), which is slightly overestimated by HPSEC (FIG. 21).

### Example 11: NMR analysis of CPS-400

The structural characteristics of CPS-400 were analyzed in a manner similar as in the examples (Table 4).

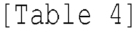

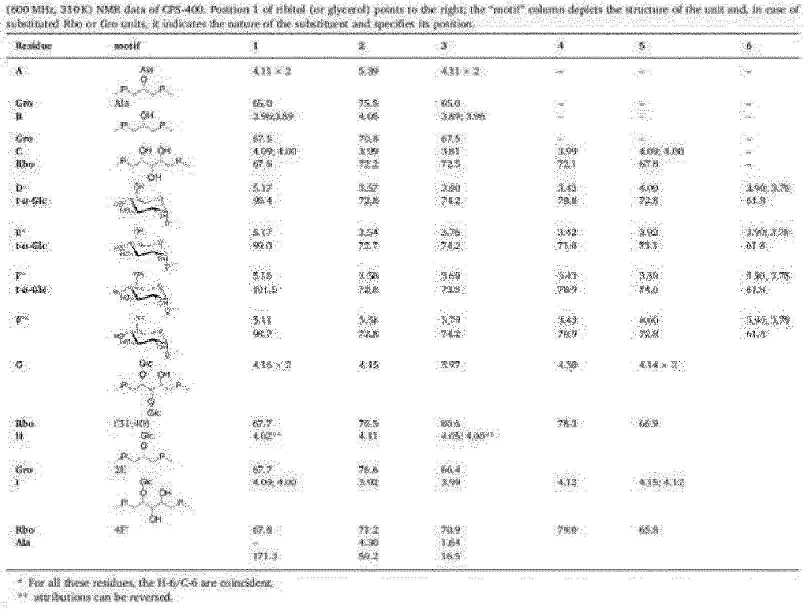

First, the anomeric regions of the HSQC spectrum (FIGS. 22A and 22B) exhibited several residues, signals at ¹H 5.3-5.1 ppm were derived from monosaccharide residues, whereas signals at ¹H/¹³C 5.39/75.5 ppm were not anomeric, but was C-2 of the glycerol unit (Gro) denoted by A, which was shifted to the lower field by acylation. The substituent at O-2 was alanine (Ala), which was identified by methyl found at ¹H/¹³C 1.64/16.5 ppm and Hα/Ca at 4.30/50.2 ppm. In addition, H-1/C-1 and H-3/C-3 of A were equivalent and were identified at 4.11/65.0 ppm based on the corresponding HSQC-TOCSY (FIG. 22A) and TOCSY (FIG. 22G) correlations.

Finally, for the C-1 (or C-3) value, the second 1,3-diphosphorylated Gro unit (B), and 1,5-diphosphorylated ribitol unit (Rbo, C) were identified in HSQC analysis performed in reference with Gerlach et al., 2018, showing that A is phosphorylated at both ends, and this last residue indicates the presence of another teichoic acid based on the ribitol-phosphate backbone. For the monosaccharide units, analysis focused on the strongest signals (D, E, F' and F) identified as α-glucose units based on the efficient propagation of magnetization up to H-6 in the anomeric signal of the TOCSY spectrum. These Glc units were further substituted based on the similarity of ¹³C chemical shifts (Bock & Pedersen, 1983).

The locations of these units were inferred through HMBC spectral analysis and comparison with reference data. Indeed, E is linked to O-2 of Gro unit (H) (Shashkov, Potekina, Senchenkova, & Kudryashova, 2009), while F' is linked to O-4 of Rbo unit (I) (Streshinskaya et al., 2011). For D, H-1 had a long-distance correlation between carbon at 78.3 ppm (FIG. 18B) and a proton (4.30 ppm) linked to "CH₂" at 69.9 ppm in the HSQC-TOCSY spectrum (FIG. 23). These new units were designated "G", and the densities at ¹H/¹³C 4.30/78.3 and 4.14/66.9 ppm were assigned as G4 and G5 (FIGS. 18C and 23). G was identified as ribitol and other signals were found by HSQC-TOSCY spectral analysis. Indeed, the "CH₂" density at ¹H/¹³C 4.16/67.7 ppm had three correlations with the signal corresponding to G4, and this density was designated "G1". In addition, C-1 (67.7 ppm) of G represents a carbon that is not glycosylated and phosphorylated at the adjacent position, as reported for I. This information led to the assignment of the remaining two HSQC-TOSCY correlations as C-2 (70.5 ppm) and C-3 (80.6 ppm), which in turn identification of H-2 (4.15 ppm) and H-3 (3.97 ppm) corresponding thereto in the HSQC spectrum (FIG. 18, FIG. 23, Table 4). Thus, G is Rbo glycosylated at O-3 and O-4, with the two units attached, F and D (HMBC in FIG. 22B).

Substitutions of this type have been identified in other *Lactobacillus plantarum* strains, but NMR data thereof report phosphate-free Rbo units, so these chemical shifts cannot be compared with our data (Tomita et al., 2017). However, the results were similar to those of Bifidobacterium teichoic acid (Valueva et al., 2013), which suggested an inverse substitution pattern of ribitol with glucose units linked to C-2 and C-3. Interestingly, the NMR data of the dephosphorylated form reported by Valueva et *al.* (2013) was consistent with the NMR data of 3,4-diglucosylated ribitol by Tomita *et al.* (2009). Therefore, the substitution pattern (2,3 or 3,4) of these ribitol units has not yet been clearly defined. Thus, the NMR data of the present invention indicate that CPS-400 is a mixture of Gro- and rbo-type two teichoic acids, each showing the presence of several substituents in a non-stoichiometric manner. For Gro-type TA, the non-stoichiometric substituents were alanine and α-glucose. For Rbo-type TA, α-glucose did not occur at both O₃ and O₄ of ribitol, O₄ alone or in any position. Separation of the two TAs was attempted by size exclusion chromatography, but CPS-400 appeared as a symmetrical peak of about 45 kDa (FIG. 21) and was not further separated.

### Example 12: Immune stimulation activity of CPS-100 and CPS-400

The activity of the CPS-100 and CPS-400 to the immune response was determined.

The effect of CPS on the immune system was determined using spleen cells in which all immune cells were mixed in a physiological ratio. Endpoint analysis was performed by testing different cytokines by ELISA. Cytokines refer to a group of secreted peptides/glycoproteins involved in cell signaling that mediates and regulates inflammatory or tolerogenic immune responses *in vivo.* Thus, distortion of cytokines in the immune cell pool upon exposure to CPS implies a similar role *in vivo.* To determine the immune response generated by CPS-100 and CPS-400, interferon gamma (IFN-γ) was analyzed as an inflammatory marker and interleukin-10 (IL-10) as a regulatory cytokine. Research results show that CPS-100 is immunostimulatory as shown by production of a high amount of IFN-γ and a negligible amount of IL-10 (FIGS. 24A and 24B), whereas no levels of IFN-γ were detected in the TA fraction, CPS-400 (FIG. 24A). Under similar conditions, other cytokines including TNF-α (tumor necrosis factor-α), IL-6 (interleukin 6), IL-12 (interleukin 12), IL-17 (interleukin 17) and IL1-β (interleukin 1β) were evaluated.

CPS-100 stimulated the cells to produce significantly higher levels of TNF-α, IL-6, and IL-12 (FIGS. 24C to 24E), whereas IL-17 and IL1-β were not detected. On the other hand, CPS-400 did not exhibit a significant increase in any of the measured cytokines (FIGS. 24C and 24E). Since IFN-γ is a primary immune stimulation marker produced by various types of immune cells, in order to determine the specificity of the immune stimulation response of CPS, whether or not the production of IFN-γ is dependent on the concentration of CPS-100 was investigated. The half maximal effective concentration (EC₅₀) of CPS-100 in IFN-γ induction for 48 hours was 3.16 µM (FIG. 24f), indicating that CPS-100 can be used as an effective immune stimulant.

The result showed that CPS-100 exhibits immunostimulatory properties similar to those of the *L. plantarum* IMB19 strain, which means that CPS-100 is an effective molecule exhibiting the immune enhancing activity of the *L. plantarum* IMB19 strain.

### Example 13: CD8+ T cell function improvement and anti-tumor immunity effects of L. plantarum IMB19 and CPS

Whether or not CPS activity as an immune stimulant *in vitro* can lead to tumor suppression activity *in vivo* was determined. It was found that the orally administered *L. plantarum* IMB19 and intraperitoneally administered CPS groups significantly reduced the growth of subcutaneous melanoma (FIGS. 25A and 25B). Delay in tumor growth in both groups was associated with infiltration of CD8+ T cells (FIG. 25C). The increase in the production and frequency of IFN-γ by tumor-infiltrating CD8+ T cells means that the cytotoxic activity of CD8+ T cells was greatly improved by the administration (FIGS. 25E and 25F). Intratumoral CD4+ T cells also exhibited upregulation of IFN-γ production in both administration groups (FIGS. 25G and 25H). On the other hand, there was no difference in Treg population in the tumor compared to the PBS administration group (FIGS. 26A and 26B). Oral administration of *L. plantarum* IMB19 regulated tumor growth in EMT-6 breast cancer (FIG. 27). Consequently, these data suggest that CPS and *L. plantarum* IMB19 enhance anti-tumor immune activity, thus inhibiting cancer growth.

### Example 14: Increased intratumoral macrophage infiltration of CPS

CPS increases macrophage infiltration in tumors. To identify the specific APC types that modulate the CD8+ T cell response, along with intraperitoneal administration of CPS, B16.F10 melanoma cells were implanted and the infiltration of APCs in the primary tumor was evaluated. CPS primarily increased the frequency of CD11c+CD11b+ macrophages in tumors compared to CD11c+DC (FIG. 28A). The results of flow cytometry showed that the number of macrophages was significantly higher in the mice administered CPS (FIG. 28A). Under the same conditions, activation markers of CD11c+CD11b+ macrophages and CD11c+ dendritic cells were confirmed. Surprisingly, the activation status of CPS-treated dendritic cells was not significantly different from that of the control group (FIG. 28C). However, macrophages were more activated and showed higher expression of CD11b, MHC I, MHC II, CD86 and CD40 (FIG. 28B). To confirm activation of the systemic adaptive immune system in the same mouse, CD69, a CD8+ T cell early activation marker, was observed in the draining lymph nodes. Compared to the control group, CD69 was clearly and significantly up-regulated by CPS administration (FIG. 28D). These data imply that CPS plays an important role in the activation of macrophages and can limit tumor growth.

### Example 15: Differentiation of macrophages into inflammatory macrophages by CPS and reprogramming of M2 macrophages to M1 phenotype

To characterize the effect of CPS on macrophages, phenotypic changes were observed when macrophages were exposed to CPS. Peritoneal CD11b+ F4/80+ macrophages exhibited an activated phenotype when treated with CPS, and significant upregulation of MHC I, MHC II, CD68, iNOS2, and CD40 in CPS-treated macrophages compared to those treated with LPS or Pam3CSK4 (FIG. 28E), indicating an M1 phenotype or an inflammatory phenotype of macrophages. In particular, similar to Pam3CSK4, TLR2 was upregulated upon CPS treatment, indicating that CPS may be a TLR2 ligand. However, in contrast to the *in vivo* experiments, the expression of CD80 and CD86 was not altered (FIG. 28E).

Alternatively, activated macrophages or M2 phenotype macrophages contribute significantly to immunosuppression and enhance tumor growth (Ann Oncol 28, xii18-xii32 (2017)) (Front Oncol 9, 421 (2019)). Thus, the results indicate that CPS in tumors can reprogram M2 macrophages to M1 phenotypes. Indeed, IL-4-induced M2 phenotype peritoneal macrophages were significantly increased when treated with CPS compared to LPS and Pam3CSK4 (FIG. 28F). In addition, the inflammatory macrophage marker iNOS2 was significantly upregulated independently of MHC I, MHC II, CD40 and CD68 (FIG. 28F). These data mean that treatment with CPS generates inflammatory macrophages and reprograms immunosuppressive macrophages to an immunostimulatory phenotype.

### Example 16: Identification of gene expression profile of CPS-modulated tumor-infiltrating macrophages

Macrophages initially infiltrated in tumors of CPS and PBS-administered mice were isolated and mRNA sequencing was performed for gene expression profiling. Several genes related to cellular chemotaxis, inflammatory response and iron ion homeostasis (FIG. 29A) were up-regulated in CPS group macrophages. The regulation of iron in the body is a physiological role primarily performed by macrophages to maintain iron sequestration and availability. Genes involved in intracellular iron ion sequestration from macrophages in the tumor microenvironment were found to be significantly upregulated (FIG. 29B). Also, Tlr2 and Tlr6 were significantly upregulated, but the gene expression level of Tlr6 was lower than that of Tlr2 (FIG. 29C) . To support the effect of CPS on macrophages, upregulation of genes representing the M1 or M2 phenotype was observed. The result showed significant upregulation of M1 inflammatory genes and significant downregulation of M2 genes (FIG. 29D), which means that Tlr2 and iron sequestration plays an important role in regulation of the inflammatory macrophage phenotype by CPS.

### Example 17: TLR2-mediated stimulation of CD8+T- cells by CPS.

To determine the role of TLRs in CPS-mediated CD8+ T-cell stimulation, the effect of DC-specific MyD88 ablation on CD8+ T-cell activation was investigated. In a co-culture experiment using MyD88-deficient dendritic cells and wild-type mouse-derived CD8+ T cells, a significant decrease in the proportion of IFN-γ cells was observed (FIG. 30A). Next, the roles of TLR2, TLR4, and TLR6 were found under similar conditions and it was found that TLR2-knockdown dendritic cells in CPS or *L. plantarum* IMB19-treated cells significantly suppressed activation of CD8+ T-cells (FIGS. 29E and 30B). This is consistent with RNA sequencing data showing TLR2 upregulation (FIG. 29C), which indicates that TLR2 plays an important role in CPS-mediated priming of CD8+ T cells.

### Example 18: Iron sequestration by CPS in tumor-associated macrophages

To determine the role of iron sequestration by macrophages in relation to anti-tumor immunity, tumor-associated macrophages were isolated from melanoma tumor-bearing mice and total intracellular soluble iron (Fe++) and iron uptake by these macrophages were tested. Total immune cells isolated from tumors were stored in iron-supplemented medium for 30 minutes or Fe++ ions were directly stained and analyzed by flow cytometry. Macrophages isolated from CPS-administered mice had high intracellular labile iron levels (FIG. 29F). In addition, iron uptake by macrophages isolated from mice administered CPS was remarkably high (FIG. 29G) . Intracellular iron levels per cell were higher in the CPS-treated group as reflected in MFI (FIGS. 29F and 29G). Therefore, iron sequestration in CPS-primed macrophages could be an important mechanism to inhibit tumor growth.

### Example 19: Intratumoral CD8+ T cell TCR repertoire regulation of CPS or L. plantarum IMB19

Whether or not CPS or *L. plantarum* IMB19 upregulates the TCR repertoire of tumor-infiltrating CD8+ T-cells associated with an increased cytotoxic response was determined. It was found that, compared to the PBS group (FIG. 31D), a specific TCR was enriched when treated with CPS or *L. plantarum* IMB19 (FIG. S14A). Simpson's diversity index was reduced upon treatment with *L. plantarum* IMB19 and CPS, causing clonal expansion of fewer selected clones compared to PBS, which exhibited stronger polyclonal response (FIG. 31B).

Pielou evenness or Shannon equitability indicates that CPS treatment increases the abundance of specific TCRs compared to PBS, which exhibits a uniform abundance of all TCRs (FIG. 31C). Thus, both CPS and *L. plantarum* IMB19 affect the TCR repertoire within the tumor microenvironment and clearly exhibit increased phenotypes of specific TCR sequences, including expression of some unique TCRs.

### Example 20: Co-administration of CPS or L. plantarum IMB19 with anticancer drugs

Experiments using co-administration were conducted in the same manner as above in order to demonstrate that *L. plantarum* IMB19 and the polysaccharide significantly inhibit tumor progression in the B16F10 mouse melanoma model and to determine the effects of the combination of *L. plantarum* IMB19 and immuneanticancer drugs on the tumors, as can be seen from the examples.

A mouse syngeneic mouse melanoma model was constructed by subcutaneous administration of 0.2 million B16F10 cells/mouse. *L*. *plantarum* IMB19 (1×10⁹ CFU/mouse) was orally administered to the mice from day 0, and an anti-PD-Ll (100 µg i.p.) antibody was administered thereto on days 7, 10, 13, and 16 (FIG. 32A). The tumor growth rate was significantly lower in the group coadministered the *L. plantarum* IMB19 strain compared to the group administered the anti-PD-Ll antibody alone (FIG. 32B).

Synergy was evaluated in a mouse kidney cancer model using the therapy against *L. plantarum* IMB19 (FIG. 32C). Renal cell carcinoma (RCC) is a cancer with an estimated 400,000 cases worldwide and 40,000 RENCA-luciferase cells were surgically implanted into the renal capsule of the right kidney of the mouse. Mice were randomly selected and treatment thereof started on day 7 (FIG. 32C).

Mice were randomly selected and treatment thereof started on day 7 (FIG. 32C) . Bioluminescence images (BLI) were obtained using IVIS spectra (Ami-HTX, Spectral Instruments Imaging, USA) at different time points to determine tumor progression.

The scale was manually set based on the last day of analysis to normalize the bioluminescence intensity at all time points. A significant synergistic effect was observed between *L. plantarum* IMB19 and anti-PD-Ll in longitudinal evaluation of tumor progression (FIGS. 32D to 32E). These results indicate that *L*. *plantarum* IMB19 can significantly enhance the anti-tumor effect of anti-PD-Ll in both therapeutic and semi-therapeutic regimens.

### [Accession number]

Name of Depositary Institution: Korea Research Institute of Bioscience and Biotechnology
Accession number: KCTC14337BP
Deposition date: 20201021

### Industrial applicability

The novel *Lactobacillus plantarum* IMB19 strain KCTC 14337BP and the polysaccharides derived from the strain of the present invention have excellent ability to stimulate CD8+ T cell activity and inhibitory activity against Treg cells, and stimulates and enhances antitumor immune responses through various mechanisms, such as increased macrophage infiltration in CPS tumors, and differentiation/reprogramming of macrophages to an inflammatory (M1) phenotype.

In particular, when the strain and polysaccharide are administered in combination with an anticancer agent, especially an immune anticancer agent, such as a PD-1 or PD-L1 inhibitor (e.g., PD-1 antibody or PD-L1 antibody), an anti-tumor immune response can be greatly improved. Therefore, the composition containing the strain and/or polysaccharide of the present invention is useful for prevention, amelioration or treatment of tumors when administered in combination with various anticancer agents.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### Sequence Free Text

An electronic file is attached.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO: MMUNOBIOME INC.,
POSTECH RESEARCH AND BUSINESS
DEVELOPMENT FOUNDATION

Cheongam-ro, Nam-gu Pohang-si,
Gyeongsangbuk-do, Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR : | Accession number given by the
INTERNATIONAL DEPOSITARY AUTHORITY:
OF DEPOSITOR
Lactobacillus plantarum IMB19 | KCTC 14337BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on October 21, 2020
IV. RECEIPT OF REQUEST FOR CONVERSION
The microorganism identified under I above was received by this International Depositary Authority
and a request to convert the original deposit to a deposit under the Budapest Treaty was received by it.
V. INTERNATIONAL DEPOSITARY AUTHORITY
Name : Korean Collection for Type Cultures | Signature(s) of person(s) having the power
to represent the International Depositary
Authority or of authorized official(s):
Address : 181 Ipsingil, Jeongeup-si Jeollabuk-do, 56212,
Republic of Korea (KRIBB) | Song-Gun Kim, Director
| Date: October 21, 2020

I certify that the above translation is the same as the above. Patent Attorney LEE, Cheo Young
Form BP/4(KCTC Form 17) Sole page

## Claims

1. A composition for preventing or treating tumors comprising a *Lactobacillus plantarum* IMB19 strain KCTC14337BP, the composition being administered in combination with an anticancer agent.

2. The composition according to claim 1, wherein the anticancer agent is an immuno-anticancer drug.

3. The composition according to claim 2, wherein the immuno-anticancer drug is an immune checkpoint inhibitor.

4. The composition according to claim 3, wherein the immune checkpoint inhibitor inhibits or blocks immune checkpoint proteins selected from the group consisting of PD-1/PD-L1, CTLA-4, IDO, B7-1 and B7-2.

5. The composition according to claim 1, wherein the tumor is selected from the group consisting of histiocytoma, glioma, astrocytoma, osteoma, various cancers, including lung cancer, small cell lung cancer, stomach cancer, gastrointestinal cancer, bowel cancer, colon cancer, rectal cancer, pancreatic cancer, breast cancer, skin cancer, ovarian cancer, prostate cancer, testicular cancer, liver cancer, kidney cancer, bladder cancer, pancreatic cancer, and brain cancer, sarcoma, osteosarcoma, melanoma, lymphoma (including Hodgkin's lymphoma, FL, MCL, MZBL, CLL, T-ALL, AML, and ALL), blood cancer, leukemia, psoriasis, bone disease, fibroproliferative disorder, and atherosclerosis.

6. The composition according to claim 1, wherein the composition is a pharmaceutical composition or a food composition.

7. A composition for preventing or treating tumors comprising a polysaccharide represented by the following Formula I as an active ingredient,
the composition being administered in combination with an anticancer agent:
[Formula I] -[-D-B-I-F-G-C-E-H-A-]ₙ-
-wherein
A and D are galactose;
B, C, E, G and H are rhamnose;
F is N-acetylglucosamine;
I is glucose; and
n is an integer from 1 to 10.

8. The composition according to claim 7, wherein at least one galactose of A or D of Formula I is phosphorylated.

9. The composition according to claim 8, wherein a hydroxyl group (-OH) of carbon 1 in A is phosphorylated.

10. The composition according to claim 8, wherein a hydroxyl group (-OH) of carbon 6 in D is phosphorylated.

11. The composition according to claim 7, wherein n in Formula I is 2 or more, and A and D of a repeating unit are linked via a phosphodiester linkage.

12. The composition according to claim 7, wherein in Formula I, D and B, and B and I are linked by an α-1,3-glycosidic linkage, I and F are linked by a β-glycosidic linkage, F and G, G and C, C and E, and E and H are linked by an α-1,2-glycosidic linkage, and H and A are linked by an α-1,6-glycosidic linkage.

13. The composition according to claim 7, wherein the polysaccharide has a structure of the following Formula II: 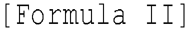 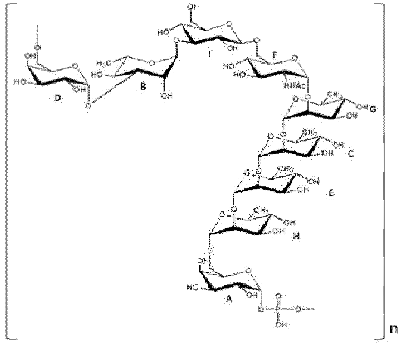 wherein n is an integer from 1 to 10.

14. The composition according to claim 7, wherein the anticancer agent is an immuno-anticancer drug.

15. The composition according to claim 14, wherein the immuno-anticancer drug is an immune checkpoint inhibitor.

16. The composition according to claim 15, wherein the immune checkpoint inhibitor inhibits or blocks immune checkpoint proteins selected from the group consisting of PD-1, PD-L1, CTLA-4, IDO, B7-1 and B7-2.

17. The composition according to claim 7, wherein the tumor is selected from the group consisting of histiocytoma, glioma, astrocytoma, osteoma, various cancers, including lung cancer, small cell lung cancer, stomach cancer, gastrointestinal cancer, bowel cancer, colon cancer, rectal cancer, pancreatic cancer, breast cancer, skin cancer, ovarian cancer, prostate cancer, testicular cancer, liver cancer, kidney cancer, bladder cancer, pancreatic cancer, and brain cancer, sarcoma, osteosarcoma, melanoma, lymphoma (including Hodgkin's lymphoma, FL, MCL, MZBL , CLL, T-ALL, AML, and ALL), blood cancer, leukemia, psoriasis, bone disease, fibroproliferative disorder, and atherosclerosis.
